# EUROPEAN PATENT SPECIFICATION

(11) **EP 2 149 614 B1**
(45) Date of publication and mention of the grant of the patent: **02.02.2011**
(21) Application number: 09171694.4
(22) Date of filing: 29.07.2003
(51) Int. Cl.: C12Q 1/68

(54) **Primers for the detection of Mycobacterium tubeculosis**
Primer zum Nachweis von Mycobacterium tuberculosis
Amorces pour la détection de Mycobacterium tuberculosis

(43) Date of publication of application: 03.02.2010
(62) Divisional of application: 03817620.2
(73) Proprietor: All India Institute of Medical Sciences, New Delhi 110 029, Maharasutra (IN)
(72) Inventor: Tyagi, Jaya Sivaswami, 110 029 New Delhi (IN); Chakravorty, Soumitesh, 110 029 New Delhi (IN)
(74) Representative: Thomas, Simon

(56) References cited:
- WO-A2-02/48391
- CHAKRAVORTY S ET AL: "Novel use of guanidinium isothiocyanate in the isolation of Mycobacterium tuberculosis DNA from clinical material" FEMS MICROBIOLOGY LETTERS, BLACKWELL PUBLISHING, AMSTERDAM, NL, vol. 205, no. 1, 27 November 2001 (2001-11-27), pages 113-117, XP002275687 ISSN: 0378-1097
- DASGUPTA N ET AL: "CHARACTERIZATION OF A TWO-COMPONENT SYSTEM, DEVR-DEVS, OF MYCOBACTERIUM TUBERCULOSIS" TUBERCLE, LONGMAN GROUP UK LTD., HARLOW, GB, vol. 80, no. 3, 1 January 2000 (2000-01-01), pages 141-159, XP009024893 ISSN: 0041-3879
- SINGH K K ET AL: "COMPARISON OF IN HOUSE POLYMERASE CHAIN REACTION WITH CONVENTIONAL TECHNIQUES FOR THE DETECTION OF MYCOBACTERIUM TUBERCULOSIS DNA IN GRANULOMATOUS LYMPHADENOPATHY" JOURNAL OF CLINICAL PATHOLOGY, BMJ PUBLISHING GROUP, vol. 53, no. 5, 1 May 2000 (2000-05-01), pages 355-361, XP009024892 ISSN: 0021-9746
- DASGUPTA N ET AL: "Mycobacterium tuberculosis DevR (devR) gene, complete cds; and DevS (devS) gene, partial cds" EMBL, 12 March 1999 (1999-03-12), XP002394848

## Description

### Field of the present invention

A multipurpose diagnostic methodology for bacterial infections including tuberculosis is described that includes processing of clinical specimens and the utilization of the processed material for smear microscopy, culture and polymerase chain reaction. It is applicable for all types of pulmonary and extra pulmonary tuberculosis.

### Background and prior art references of the present invention.

Tuberculosis kills more people in India and South-East Asia than any other infectious disease - more than HIV, STD, malaria and tropical diseases combined. In India more than one thousand people die from TB everyday - more than 450,000 per year, 1 every minute. Rapid diagnosis of tuberculosis is becoming increasingly important to improve cure rates, reduce the risk of spreading the disease through pulmonary route, and to combat the recent emergence of drug resistant strains of *M. tuberculosis* bacteria and its severe implications in HIV-infected patients. The causative organism of the disease is *Mycobacterium tuberculosis.* This organism is characterized by its slow growth and the presence of a unique lipid-rich cell wall that makes it unamenable to the action of conventional antibiotics and lytic procedures. The acid-fast properties of the cell wall are utilized to rapidly detect the presence of *M. tuberculosis* and other mycobacteria in clinical specimens by smear microscopy.

By far smear microscopy is the most popular amongst all methods currently employed worldwide in the laboratory diagnosis of tuberculosis on account of its simplicity, speed, low cost and minimal requirement for equipment and technical skill. However it suffers from lack of sensitivity since a load of ∼10,000 organisms/ml of specimen is required to give a positive report by Ziehl Neelsen staining from concentrated samples (Kent and Kubica, 1995). A more sensitive smear microscopy method that is also simple would be highly useful in diagnostic laboratories. Culture is considered as the gold standard, but on account of the slow growth rate of the pathogen, sole dependence on it would invariably lead to a delay of 4-6 weeks in arriving at a definitive diagnosis. Apart from pulmonary tuberculosis, extra pulmonary tuberculosis presents an enormous diagnostic challenge on account of the wide spectrum of organs involved and the paucibacillary load of bacteria in the sample sent to the diagnostic laboratory.

Nucleic acid amplification techniques were championed as providing alternative sensitive, specific and rapid approaches to tuberculosis diagnosis. These approaches have suffered in no small measure from the inability to isolate mycobacterial PCR-amplifiable DNA of adequate quality from all types of clinical material. To the best of our knowledge a DNA isolation method using environment-friendly reagents that is universally applicable on all types of clinical material is currently not available. This presents a very practical and serious impediment to the application of DNA amplification methodologies to clinical material.

Against this background, a single universal methodology has been developed which includes a unique sample processing technique that alone is compatible with the most commonly used laboratory methods of TB diagnostics, namely, smear microscopy, culture and PCR. It is applicable on all types of clinical material except fecal matter, in pulmonary and extra pulmonary cases of tuberculosis. The method takes advantage of the unique properties of the mycobacterial cell wall to selectively remove extraneous materials, contaminating organisms and potential PCR-inhibitory material without adverse effect on *M. tuberculosis* acid-fast properties, viability and integrity. The chaotropic properties of guanidinium hydrochloride are utilized for this purpose in conjunction with detergents and neutral pH buffer.

Smear microscopy and culture are the most widely used laboratory techniques for the diagnosis of tuberculosis worldwide. In recent years, nucleic acid-based approaches, PCR in particular, are also being employed as an adjunct test to arrive at a definitive diagnosis on a reasonable period of time. To the best of our knowledge, published techniques and commercial kits for smear microscopy, culture and PCR along with their shortcomings are as follows:
***Smear microscopy and culture:*** The methods for preparing samples for smear microscopy and cultivation of *M. tuberculosis,* involve the use of 4% NaOH; 0.5% NALC and 2% NaOH (3% NaOH is recommended for tropical countries with highly humid climates);
dithiothreitol (DTT) and 2% NaOH; 13% trisodium phosphate with or without benzalkonium chloride (Zephiran); 5% oxalic acid; 1% cetylpyridium chloride and 2% NaCl. (Koneman *et al.* 1997). Household bleach (NaOCl) has also been used for liquefaction of sputum and preparation of smears. It requires overnight precipitation and
smearing from sediment. In 3 studies performed in Ethiopia and India, the use of the NaOCl method increased the number of samples positive for acid-fast bacilli by more than 100% (Gebre *et al.* 1995).

But some studies have demonstrated that though bleach sedimentation can increase the diagnostic yield, it is only to a minor extent (Van Deun *et al.* 2000). The basic aim of each of the methods is to liquefy and decontaminate the sample and have been most widely used for sputum samples. NALC and DTT are effective mucolytic agents but are costly. Decontaminants like NaOH and trisodium phosphate require that the exposure time be controlled carefully. Zephiran requires neutralization with lecithin and is not compatible with inoculation on egg-based culture media. (Koneman. *et al.,* 1997).

Recently a method for processing respiratory specimens by using C₁₈-Carboxypropylbetaine (CB-18^{™}), a zwitterionic detergent has been described for detection of mycobacteria. The method involves mild alkaline digestion of the sample and treatment with CB-18^{™} followed by an incubation of 2-24 hours, centrifugation and smearing for AFB. For culturing of *M. tuberculosis* a separate protocol is prescribed wherein three lytic enzymes are used in conjunction with CB -18^{™} which increases the cost. The initial study showed that the aggregate smear sensitivity and culture sensitivity increases by approximately 58 % and 43 % respectively by this method when compared to the NALC-NaOH method (Thornton *et. al.* 1998).

In a recent report where the CB-18^{™} method was compared with the NALC-NaOH method, the former method showed a smear sensitivity of 59.6 % that later increased to 71.4 % on modification of the protocol (Scott *et al.* 2002). It is to be noted that sample processing by this method is not compatible with culturing in liquid media. This product is marketed by Quest Diagnostics Incorporated, Baltimore, USA as CB-18^{™} Smear Kit and CB-18^{™} TB Culture Kit with Lytic DECON II.

Another method using chitin for mucus digestion in sputum has been described recently which involves the addition of chitin solution to the sputum, homogenization by vortexing or shaking, sedimentation at unit gravity for 30 minutes and preparation of smear from the sediment. This is suitable only for smear microscopy from sputum. The sensitivity of this method was estimated to be 80% with a specificity of 96.7% (Farnia *et al.,* 2002). The utility of this method for culture was not evaluated.

Another recently published method describes the use of phenol and ammonium sulfate combination (PhAS) for liquefaction of sputum. But this needs an overnight precipitation and is not suitable for culturing as it kills the mycobacteria. The sensitivity and specificity of the method were 85% and 97% respectively (Selvakumar *et al.* 2002).

The sensitivity of the direct method of smearing (by ZN and/or auramine staining) has ranged from 40 to 85 % in different laboratory settings. Involvement of a concentration step increased the sensitivity of the direct smear method from 54-57 % to 63-80% (Bruchfeld *et al.* 2000; Garay *et al.* 2000). The CDC method has shown sensitivity levels around 66 to 83% (Scott *et al.* 2002, Farnia *et al.* 2002, our study). Inclusion of a centrifugation step (4000 g for 15 min) was reported to detect ≥ 5000 and 500 organisms/ml by smear microscopy and culture, respectively (Perera *et al.* 1999). However other reports have suggested that the overall diagnostic sensitivity of smear microscopy was not increased by sputum liquefaction and concentration (Wilkinson *et al.* 1997). It has been proposed that the use of fluorescence microscopy greatly improves the diagnostic value of the sputum smear especially in samples with a low density of bacilli that are likely to be missed on ZN stained smears (Githui *et al.* 1993). However the fluorescence microscopy technique would increase the cost.

Automated and semi-automated systems of cultivation of Mycobacteria currently available-The most sensitive though time consuming method for diagnosis of TB is culturing of the etiologic agent. There are several liquid and solid media available in the international market for this purpose. Since growth in a medium like Lowenstein-Jensen or other solid media takes at least 4-6 weeks to arrive at conclusive result, liquid culture systems employing radioactive materials or oxygen quenching and redox reagents have been introduced for their superior speed and sensitivity. They all require respiratory specimens that have been decontaminated by treatment with NALC-NaOH. The most widely used *M.tb* culturing kits based on these principles are: 1). BACTEC 460 TB (Becton Dickinson Diagnostic Instruments, Sparks, MD). It detects consumption of CO₂ radiometrically, 2). MB/BacT (Organon Teknika Corporation. Durham NC). It detects consumption of CO₂ colorimetrically. 3). ESP Myco System (Difco Laboratories, Detroit, Michigan). It detects change in gas pressure. 4).

Mycobacterial Growth Indicator Tube (MGIT): Becton Dickinson, Cockeysville, MD). It detects consumption of oxygen through an oxygen-sensitive fluorescent sensor. 5). Septi-Check AFB System (BBL): Colonies are detected visually. All of the above systems use liquid culture media, (except for Septi-Check which is a biphasic system having both liquid and solid media) growth supplements, antibiotics and involve sophisticated technology. Except for the Septi-Check AFB systems the other systems are automated and results can be obtained as early as 5-7 days. But with these automated systems it is not possible to study the colony morphology of the cells growing and the absence of contaminants have to be confirmed by performing AFB staining of the positive cultures. These systems are very costly and out of place in disease-endemic countries like India. The radiometric detection is a biological hazard too.

DNA isolation and in-house PCR - Numerous in-house methods aimed at isolation of PCR amplifiable *M. tuberculosis* DNA have been described. But a single method that is (a) compatible with smear microscopy, culture and DNA isolation from clinical samples and (b) suitable for all kinds of pulmonary and extrapulmonary samples, is not available. All the methods require pretreatment of the sputum samples with a mucolytic agent (NALC or DTT) and/or decontamination using NaOH and invariably involve the use of organic solvents and enzymes. The most popular methods for DNA isolation to the best of our knowledge are as follows:
1. Phenol chloroform method: NALC-NaOH-treated sputum sample is further subjected to treatment with lytic enzymes and organic solvents followed by Cetyl Trimethyl Ammonium Bromide treatment and DNA precipitation with isopropanol. (Singh *et al.* 2000).
2. Detergents method: NALC-NaOH - treated sputum pellet digested with lysozyme. DNA is obtained by heating at 55°C with detergents in conjunction with proteinase K. (Perera *et al.* 1994).
3. Chelex 100 resin and detergent method: DTT-fluidified sputa are subjected to DNA extraction by adding 15% Chelex, 0.1% SDS, 1% NP40 and 1% T20 and heating for 30 mins. at 100°C (de Lamballerie *et al.* 1992). Our studies have shown that SDS is inhibitory to PCR and Triton X-100 provides better lysis than NP40 (Chakravorty and Tyagi, 2001). Chelex 100 resin has also been used to isolate *M. tuberculosis* DNA from extrapulmonary samples (Walsh *et al.* 1991).
4. Lysis of *M. tuberculosis* and capturing of DNA in silica in the presence of guanidinium isothiocyanate (GITC): Sputum, bronchial washings, pus and pleural fluid treated with NALC-NaOH and centrifuged. CSF and urine centrifuged directly. Tissues are homogenized and treated with Proteinase K-SDS for 12-16 hours. Lysis is induced by using GITC silica suspension and heating at 80°C, followed by mixing to bind the DNA, washing of silica particles and elution in water. The method is cumbersome, involves toxic GITC, tissue processing takes longer time and requires enzymatic digestion, and has been developed only for DNA isolation (Noordhoek *et al.* 1995).
5. The inventors have previously published a method of obtaining inhibitor-free, PCR- amplifiable *M. tuberculosis* DNA from pulmonary and extrapulmonary samples. The method though simple, rapid and efficient uses toxic reagent GITC, requires NALC treatment and was not assessed for compatibility with smear microscopy and culture (Chakravorty and Tyagi, 2001). Chakravorty and Tygai, 2001, also disclose primers for amplifying the dev R gene of M. tuberculosis used to detect said bacteria.

### Commercial nucleic acid detection kits.

In the international market, the following diagnostic kits employing amplification of the DNA or RNA of *M. tuberculosis* are available to the best of our knowledge. They use different amplification technologies (PCR, TMA, SDA and LCR). They require either respiratory or non-respiratory specimens that have been pretreated by NALC-NaOH (CDC method).
1. AMPLICOR® Microwell Plate and COBAS AMPLICOR^{™} Analyzer Automated PCR Tests.
   (Roche Molecular Systems, USA). The AMPLICOR® MTB Test is a PCR-based system to detect *M. tuberculosis* in untreated patients who have AFB (Acid-Fast bacilli) positive smear results. Rapid identification: 1 day, qualitative test.
   The COBAS AMPLICOR™ Analyzer is a benchtop automated system to perform the amplification and detection steps of the Polymerase Chain Reaction (PCR) testing process. It employs a single instrument that combines five instruments into one (thermal cycler, automatic pipettor, incubator, washer and reader).
2. Amplified Mt Direct Test (AMTDT I) and AMTDT II (Gen-Probe, USA). The AMTDT is based on the isothermal amplification of 582 bp segment of the 16S rRNA gene for detection of *M. tuberculosis.* This system uses the TMA method to amplify rRNA via DNA intermediates, followed by chemiluminiscent detection of amplicons with an acridinium-ester-labeled DNA probe. It involves the use of two enzymes (reverse transcriptase and RNA polymerase) and a sonicator for sample preparation. It is the first kit to be approved by FDA for use on AFB smear positive samples.
3. LCx *Mtb* test (Abbott Laboratories, USA). This test is based on the amplification by ligase chain reaction of a segment of the chromosomal gene of *M. tuberculosis* encoding for the protein antigen b. Sample preparation consists of two washes and centrifugations to remove inhibitors, heating at 90°C for 10 min to kill mycobacteria, and sonication to lyse the cells. After amplification using two enzymes (polymerase, ligase), the target is detected by Micro-particle Enzyme Immunoassay (MEDIA) automated analyzer.
4. BDProbeTec (Becton Dickinson, USA). The BDProbeTec technology basically consists of the following steps: (i) heating of the NALC-NaOH-treated samples, (ii) decontamination, (iii) amplification (SDA), (iv) hybridization, and (v) detection. Zirconium beads and silica are used for mechanical disruption of the mycobacterial cell wall in conjunction with incubation at high temperature in a Lysolyzer instrument (Becton Dickinson). Subsequent breakage employs another instrument, the FastPrep apparatus (Bio 101, Vista, USA). Specimens are then analyzed with the fully automated BDProbeTec instrument, i.e., for amplification and hybridization. Detection of the products is by a spectrophotometer provided with the BDProbeTec equipment.

In summary, these commercial technologies are very sophisticated, costly including high running costs and generally not suitable for developing countries. These countries also happen to be high-incidence countries with maximum demand for cheap, rapid and efficient diagnostic test(s).

The two most commonly employed method of smear microscopy are direct smear examination (Akhtar *et al.,* 2000) and concentration method recommended by CDC, Atlanta, U.S.A. (Koneman *et al.* 1997). Their drawbacks are enumerated below.

### Drawbacks of current methods of smear microscopy

### Drawbacks of direct smear examination:

1. The process though very rapid and relatively easy, is not sensitive. The lower limit of detection its ∼10,000 acid-fast bacilli (AFB) / ml of sputum (Kent and Kubica, 1995).
2. The process involves collecting a purulent portion of the sputum for optimal results. This needs proper training of technicians to identify the purulent portion, failure to collect which may result in false-negative smear reporting even from sputum samples containing >10,000 AFB/ml.
3. The direct smear method prefers "ideal" sputum samples which are purulent. Samples with nasopharyngeal discharge or saliva are discouraged though they may contain detectable levels of AFB.
4. Since the purulent portion of the sputum is dealt with, there are chances that the smear may be too thick which is not suitable for smear microscopy. Even with properly smeared slides, the counterstaining material (due to the mucus, protein and tissue/cell debris present in the sputum) often hinders proper reading of slides).
5. This method is primarily used on sputum samples.

### Drawbacks of concentration method (NALC-NaOH method) of smear examination:

1. The process though deals with the entire sputum sample, involves a costly reagent NALC.
2. The reagent is to be prepared fresh every time prior to sample processing.
3. The method has been developed and extensively used only for sputum samples.
4. Only one or two loopfuls of the concentrated sample is smeared which does not always allow detectable levels of AFB to be present in the smear in case of paucibacillary samples.

### Drawbacks of current method of culture:

Clinical specimens are routinely decontaminated to remove organisms other than mycobacteria prior to culture on solid and liquid media. This is necessary as mycobacteria are slow-growing and cultures are swamped by faster-growing contaminating organisms.
The most popularly used method of decontamination uses 2-3% NaOH along with the mucolytic agent NALC.
1. The CDC method (NALC/NaOH method) utilizes NaOH as a decontaminating agent that needs to be completely neutralized. Failure to do so renders the sample unsuitable for culturing.
2. The method has been developed and extensively used for sputum samples.
3. Decontamination using 2% NaOH is not always suitable for tropical countries with a humid climate where use of 3% NaOH is recommended which minimizes the recovery of live mycobacteria due to toxicity effects (Krasnow and Wayne, 1966).

### Drawbacks of currently used methods of DNA isolation for mycobacterial PCR:

1. A universal method of isolation of mycobacterial DNA from all kinds of pulmonary and extra-pulmonary samples is not available.
2. Majority of the methods of DNA isolation, either involve hazardous organic reagents or expensive enzymes.
3. There is no environment-friendly method that guarantees the isolation of PCR quality inhibitor-free mycobacterial DNA from all types of clinical samples.

### Objects of the present invention.

The main object of the present disclosure is to develop an effective and economical method of processing clinical samples useful for simple, rapid, safe, sensitive, and accurate diagnosis of tuberculosis and certain gram positive bacterial infections.

Another main object of the present disclosure is to develop the processed sample in the form of smear, culture, or Polymerase chain reaction (PCR) starting material, using PCR amplifiable mycobacterial DNA, and RNA.

Yet another object of the present invention is to develop a set of primers for screening patients for tuberculosis.

Still another object of the present disclosure is to develop a processed clinical sample.

Still another object of the present disclosure to develop a single methodology, which alone encompasses sample processing and the most common laboratory methods of TB diagnostics, namely, smear microscopy, culture and PCR/RT-PCR.

Still another object of the present disclosure to develop a methodology that is applicable on all types of clinical material in pulmonary and extra pulmonary cases of tuberculosis and not just sputum.

Still another object of the present disclosure is to enable the microscopic visualization of AFB from samples with minimal bacterial load.

Still another object of the present disclosure is to develop a decontamination process other than NaOH treatment, which can avoid the cumbersome process of neutralization of samples and also reduce toxicity.

Still another object is to eliminate the use of NALC or other costly mucolytic agents.

Still another object of the present disclosure is to have a broad-based utility in the detection, isolation and amplification of DNA from all mycobacterial species including opportunistic pathogens.

Still another object of the present disclosure is to isolate inhibitor-free PCR amplifiable DNA from a wide variety of pulmonary and extra-pulmonary samples.

Still another object of the present disclosure is to avoid the use of organic solvents and enzymes for the isolation of clean PCR-amplifiable DNA from clinical material.

Still another object of the present disclosure is to use chaotropic agents in the process that is not toxic and is environment friendly and safe to use.

Still another object of the present disclosure is to develop a simple methodology that has minimal dependence on refrigerated high-speed centrifuge and sophisticated instrumentation.

Still another object of the present disclosure is to develop a method that involves minimal transfer of sample from one tube to another during processing, thus preventing loss of sample.

Still another object of the present disclosure is to develop a method that is compatible with isolation of mycobacterial RNA from clinical specimens.

Still another object of the present disclosure is to develop a modular technology for the diagnosis of tuberculosis.

Still another object of the present disclosure is to develop a highly sensitive method for processing clinical samples to identify the disease condition.

### Summary of the present invention

An effective and economical method of processing clinical samples useful for simple, rapid, safe, sensitive, and accurate diagnosis of bacterial infections using a composition comprising solution 1 consisting of Universal Sample Processing (USP) solution (composed of Guanidinium Hydrochloride (GuHCl) of concentration ranging between 3-6M, Tris-Cl of concentration ranging between 40-60 mM of pH ranging between 7.3- 7.7, EDTA of concentration ranging between 20-30mM, Sarcosyl of concentration ranging between 0.3 - 0.8%, beta-mercaptoethanol of concentration ranging between 0.1-0.3 M), Solution 2 consisting of Sodium phosphate of concentration ranging between 65 to 70 mM of pH ranging between 6.7 to 6.8 (optional can be replaced with sterile water), and Solution 3 consisting of Tween 80 of concentration ranging between 0.03 to 0.08%, and Solution A comprising Chelex 100 suspension of concentration ranging between 8-12%, Solution B consisting of Triton X-100 of concentration ranging between 0.02-0.04%, and Solution C comprising Tween 20 of concentration ranging between 0.2-0.4% for isolating DNA (optionally, Solution 3 alone or Solution 3 with 0.03-0.1 % Triton X 100 can be used for isolating DNA from high bacillary load or low junk containing samples in place of solutions A, B and C), said method comprising steps of adding 1.5 to 2 volumes of solution 1 to the sample and homogenization, adding Solution 2 or sterile water to the homogenate and centrifuging to obtain pellet, rewashing the pellet with solution 1 (optionally, for high junk containing samples), washing the solution, 1-washed pellet with water, and resuspending the water-washed pellet in solution 3 to obtain processed sample in a from suitable for implementation of standard diagnostic procedures by smear microscopy, culture and PCR, and a Kit comprising above-stated solutions, and two sets of primers with first set devRf2 and devRr2 of SEQ ID No. 1 and SEQ ID No. 2 respectively and second set of primers devRf3, and devRr3 of SEQ ID No. 3, and SEQ ID No. 4 respectively, and a method of using said primers for screening patients of tuberculosis by polymerase chain reaction.

### Detailed description of the present invention.

Accordingly, the present disclosure relates to an effective and economical method of processing clinical samples useful for simple, rapid, safe, sensitive, and accurate diagnosis of bacterial infections using a composition comprising solution 1 consisting of Universal Sample Processing (USP) solution (composed of Guanidinium Hydrochloride (GuHCl) of concentration ranging between 3-6M, Tris-Cl of concentration ranging between 40-60 mM of pH ranging between 7.3- 7.7, EDTA of concentration ranging between 20-30mM, Sarcosyl of concentration ranging between 0.3 - 0.8%, beta-mercaptoethanol of concentration ranging between 0.1-0.3 M), Solution 2 consisting of Sodium phosphate of concentration ranging between 65 to 70 mM of pH ranging between 6.7 to 6.8 (optional can be replaced with sterile water), and Solution 3 consisting of Tween 80 of concentration ranging between 0.03 to 0.08%, and Solution A comprising Chelex 100 suspension of concentration ranging between 8-12%, Solution B consisting of Triton X-100 of concentration ranging between 0.02-0.04%, and Solution C comprising Tween 20 of concentration ranging between 0.2-0.4% for isolating DNA (optionally, Solution 3 alone or Solution 3 with 0.03-0.1 % Triton X 100 can be used for isolating DNA from high bacillary load or low junk containing samples in place of solutions A, B and C), said method comprising steps of adding 1.5 to 2 volumes of solution 1 to the sample and homogenization, adding Solution 2 or sterile water to the homogenate and centrifuging to obtain pellet, rewashing the pellet with solution 1 (optionally, for high junk containing samples), washing the solution 1-washed pellet with water, and resuspending the water-washed pellet in solution 3 to obtain processed sample in a from suitable for implementation of standard diagnostic procedures by smear microscopy, culture and PCR, and a Kit comprising above-stated solutions, and two sets of primers with first set devRf2 and devRr2 of SEQ ID No. 1 and SEQ ID No. 2 respectively and second set of primers devRf3, and devRr3 of SEQ ID No. 3, and SEQ ID No. 4 respectively, and a method of using said primers for screening patients of tuberculosis by polymerase chain reaction.

In an embodiment of the present disclosure wherein an effective and economical method of processing clinical samples useful for simple, rapid, safe, sensitive, and accurate diagnosis of bacterial infections using a composition comprising solution 1 consisting of Universal Sample Processing (USP) solution (composed of Guanidinium Hydrochloride (GuHCl) of concentration ranging between 3-6M, Tris-Cl of concentration ranging between'40-60 mM of pH ranging between 7.3- 7.7, EDTA of concentration ranging between 20-30M, Sarcosyl of concentration ranging between 0.3 - 0.8%, beta-mercaptoethanol of concentration ranging between 0.1-0.3 M), Solution 2 consisting of Sodium phosphate of concentration ranging between 65 to 70 mM of pH ranging between 6.7 to 6.8 (optional can be replaced with sterile water), and Solution 3 consisting of Tween 80 of concentration ranging between 0.03 to 0.08%, and Solution A comprising Chelex 100 suspension of concentration ranging between 8-12%, Solution B consisting of Triton X-100 of concentration ranging between 0.02-0.04%, and Solution C comprising Tween 20 of concentration ranging between 0.2-0.4% for isolating DNA (optionally, Solution 3 alone or Solution 3 with 0.03-0.1 % Triton X 100 can be used for isolating DNA from high bacillary load or low junk containing samples in place of solutions A, B and C), said method comprising steps of:
- obtaining the clinical sample,
- mixing 1.5 to 2 volumes of solution 1 to the sample,
- homogenizing the mixture while avoiding frothing,
- adding Solution 2 or sterile water to the homogenate followed by centrifugation to obtain pellet,
- washing the pellet with solution 1, optionally depending upon the decrease of the pellet size,
- washing the solution 1-washed pellet with water, and
- resuspending the water-washed pellet in solution 3 to obtain processed sample for diagnosis.

In yet another embodiment of the present disclosure, wherein the processed sample can be used in the form of smear, culture, or Polymerase chain reaction (PCR) starting material, using PCR amplifiable mycobacterial DNA, and RNA.

In still another embodiment of the present disclosure, wherein said Universal Sample Processing (USP) solution comprises Guanidinium Hydrochloride (GuHCl) of concentration ranging between 3-6M, Tris-Cl of concentration ranging between 40-60 mM of pH ranging between 7.3- 7.7, EDTA of concentration ranging between 20-30mM, Sarcosyl of concentration ranging between 0.3 - 0.8%, beta-mercaptoethanol of concentration ranging between 0.1-0.3 M.

In still another embodiment of the present disclosure wherein the processed sample can be used for smear microscopy of mycobacteria including *M. tuberculosis* and Gram-positive organisms like *Staphylococcus sp.*

In still another embodiment of the present disclosure wherein homogenizing is required for time duration ranging between 20-120 seconds.

In still another embodiment of the present disclosure, wherein Guanidinium hydrochloride of solution 1 lyses eukaryotic and Gram negative cells, denatures proteins, liquefies sample, and inactivates endogenous enzymes.

In still another embodiment of the present disclosure, wherein the processing is completed in a total time duration ranging between 1- 2 hours.

In still another embodiment of the present disclosure, wherein said method yields inhibitor-free mycobacterial DNA for PCR based diagnostics of mycobacterial diseases including TB and leprosy.

In still another embodiment of the present disclosure wherein Universal Sample Processing (USP) solution for processing only culture and smear samples comprises Guanidinium Hydrochloride (GuHCl) of concentration of about 4M, Tris-Cl of concentration ranging between 40-60 mM of pH ranging between 7.3- 7.7, EDTA of concentration ranging between 20-30mM, Sarcosyl of concentration ranging between 0.3 - 0.8%, beta-mercaptoethanol of concentration of about 0.1M.

In still another embodiment of the present disclosure, wherein said Universal Sample Processing (USP) solution for processing culture, smear, and PCR samples comprises Guanidinium Hydrochloride (GuHCl) of concentration of about 5M, Tris-Cl of concentration ranging between 40-60 mM of pH ranging between 7.3- 7.7, EDTA of concentration ranging between 20-30mM, Sarcosyl of concentration ranging between 0.3 - 0.8%, beta-mercaptoethanol of concentration ranging between 0.1-0.2 M.

In still another embodiment of the present disclosure, wherein said Universal Sample Processing (USP) solution for processing only smear, and PCR samples comprises Guanidinium Hydrochloride (GuHCl) of concentration of about 6M, Tris-Cl of concentration ranging between 40-60 mM of pH ranging between 7.3- 7.7, EDTA of concentration ranging between 20-30mM, Sarcosyl of concentration ranging between 0.3 - 0.8%, beta-mercaptoethanol of concentration of about 0.2 M.

In still another embodiment of the present disclosure, wherein Guanidinium Hydrochloride (GuHCl), Sarcosyl, and beta-mercaptoethanol of USP act in a synergistic manner to perform optimal processing of all kinds of clinical samples.

In still another embodiment of the present disclosure wherein presence of beta-mercaptoethanol and Sarcosyl is suitable for optimal processing of mucoid sputum samples.

In still another embodiment of the present disclosure, wherein presence of GuHCl is absolutely necessary along with beta-mercaptoethanol and Sarcosyl for optimal processing of mucopurulent sputum samples.

In still another embodiment of the present disclosure, wherein GuHCl acts as the principal inhibitor removal component in case of specimens containing blood, by denaturing hemoglobin and removing it from the specimen.

In still another embodiment of the present disclosure, wherein GuHCl acts as the principal decontaminating agent of clinical specimens.

In still another embodiment of the present disclosure, wherein PCR-amplifiable mycobacterial DNA can be obtained through simple lysis by boiling in presence of Solution 3 and/or 0.03-0.1 % Triton X 100, without using Solution A, B and C in case of high bacillary load and/or lesser amount of junk containing samples.

In still another embodiment of the present disclosure, wherein the processed sample is free of contaminating organisms, proteins, enzymes, and interfering substances.

In still another embodiment of the present disclosure, wherein said method under smear microscopy can detect about 300-400 bacilli/ml of the sample. The sensitivity (limit of detection) can be further increased by using more than the prescribed 10% of the processed specimen for smear microscopy.

In still another embodiment of the present disclosure, wherein said method has about 30 folds enhancement in sensitivity over the conventional direct smear microscopy method.

In still another embodiment of the present disclosure, wherein said method in a smear shows sensitivity ranging between 97-99%.

In still another embodiment of the present disclosure wherein said method in a smear shows specificity ranging between 83-92%.

In still another embodiment of the present disclosure, wherein said method shows enhancement in sensitivity by about 18% over CDC method of smear microscopy.

In still another embodiment of the present disclosure, wherein said method shows enhancement in sensitivity by about 30% over direct smear method.

In still another embodiment of the present disclosure wherein said method in smears shows positive predictive value ranging between 80-96%.

In still another embodiment of the present disclosure, wherein said method in smears shows negative predictive value ranging between 91 -99 %.

In still another embodiment of the present disclosure, wherein said method in smears shows diagnostic accuracy of about 91%.

In still another embodiment of the present disclosure, wherein said method reduces counterstaining background enabling better viewing of the slides, enhances the gradation of slides, and reduces time and labour for reading a slide.

In still another embodiment of the present disclosure wherein said method carries out decontamination of samples more efficiently as compared to the CDC method.

In still another embodiment of the present disclosure, wherein said method in a culture leads to a lag of about 1 week in appearance of the microbial colonies as compared to untreated microbes.

In still another embodiment of the present disclosure, wherein said method in a culture shows sensitivity of about 50%.

In still another embodiment of the present disclosure wherein said method is more suitable than CDC method in tropical areas.

In still another embodiment of the present disclosure wherein said method in culture runs at a neutral pH.

In still another embodiment of the present disclosure, wherein said method in PCR shows no inhibition of PCR assay with any type of clinical specimens tested.

In still another embodiment of the present disclosure, wherein said method in PCR shows sensitivity ranging between 96.5-100%.

In still another embodiment of the present disclosure, wherein said method in PCR shows specificity ranging between 71-100%.

In still another embodiment of the present disclosure, wherein said method in PCR using said primers shows positive predictive value ranging between 82-100%.

In still another embodiment of the present disclosure, wherein said method in PCR using said primers shows negative predictive value of 94-100%.

In still another embodiment of the present disclosure, wherein said method in PCR using said primers shows diagnostic accuracy of about 90%-100%.

In still another embodiment of the present disclosure, wherein samples can be obtained from sources comprising all types of sputum and other body fluids comprising FNAC, pus, pleural fluid, pericardial fluid, joint aspirate, peritoneal fluids, cerebrospinal fluids, endometrial aspirate, synovial fluid, gastric aspirate, endotracheal aspirate, urine, transtracheal aspirate, bronchoalveolar lavage, laryngeal swab and nasopharyngeal swab; body tissues comprising blood, pleural tissue, bone marrow and biopsy; solid organs comprising lymph node, bone, skin, and bovine samples comprising lymph gland, milk, and blood.

In still another embodiment of the present disclosure, wherein samples stored at about - 20°C for upto 2 months can be processed for PCR, smear-microscopy and culture.

In still another embodiment of the present invention, wherein said method in PCR can be used for both DNA, and RNA.

In still another embodiment of the present disclosure, wherein said composition shows mucolytic, decontaminating, protein denaturant, chaotropic, liquefying, tissue softening/digesting, and mycobacteria-releasing action.

In still another embodiment of the present disclosure, wherein said method in smear enables more bacilli to be smeared on the slide thereby increases both sensitivity, and efficiency.

In still another embodiment of the present disclosure, wherein said method in smear generally converts slides that are graded as 1+ or scanty by the direct method to 2+/3+ or 2+/1+ respectively.

In still another embodiment of the present disclosure wherein said method helps process sputum samples lacking purulence or containing nasopharyngeal discharge and/or saliva and/or blood.

In still another embodiment of the present disclosure, wherein said method yields high quality smears from tissue biopsy samples suitable for very sensitive AFB smear microscopy.

In another main embodiment of the present disclosure, wherein an effective and economical method of processing clinical samples useful for simple, rapid, safe, sensitive, and accurate diagnosis of Mycobacterial infections especially *tuberculosis* caused by *Mycobacterium tuberculosis* using a composition comprising solution 1 consisting of Universal Sample Processing (USP) solution (composed of Guanidinium Hydrochloride (GuHCl) of concentration ranging between 3-6M, Tris-Cl of concentration ranging between 40-60 mM of pH ranging between 7.3- 7.7, EDTA of concentration ranging between 20-30mM, Sarcosyl of concentration ranging between 0.3 - 0.8%, beta-mercaptoethanol of concentration ranging between 0.1-0.3 M), Solution 2 consisting of Sodium phosphate of concentration ranging between 65 to 70 mM of pH ranging between 6.7 to 6.8 (optional can be replaced with sterile water), and Solution 3 consisting of Tween 80 of concentration ranging between 0.03 to 0.08%, and Solution A comprising Chelex 100 suspension of concentration ranging between 8-12%, Solution B consisting of Triton X-100 of concentration ranging between 0.02-0.04%, and Solution C comprising Tween 20 of concentration ranging between 0.2-0.4% for isolating DNA (optionally, Solution 3 alone or Solution 3 with 0.03-0.1 % Triton X 100 can be used for isolating DNA from high bacillary load or low junk containing samples in place of solutions A, B and C), said method comprising steps of:
- obtaining the clinical sample,
- mixing 1.5 to 2 volumes of solution 1 to the sample,
- homogenizing the mixture while avoiding frothing,
- adding Solution 2 to the homogenate and centrifuging to obtain pellet, optionally solution 2 can be replaced with sterile water,
- washing the pellet with solution 1,
- washing the solution 1-washed pellet with water, and
- resuspending the water-washed pellet in solution 3 to obtain processed sample for diagnosis.

In still another embodiment of the present disclosure, wherein the processed sample can be used in the form of smear, culture, or Polymerase chain reaction (PCR) starting material using PCR amplifiable mycobacterial DNA, and RNA.

In still another embodiment of the present disclosure, wherein the processed sample can be used in the form of smear, culture, or Polymerase chain reaction (PCR) starting material, wherein the PCR amplifiable mycobacterial DNA can be obtained by simple lysis by boiling.

In still another embodiment of the present disclosure, wherein said Universal Sample Processing (USP) solution comprises Guanidinium Hydrochloride (GuHCl) of concentration ranging between 3-6M, Tris-Cl of concentration ranging between 40-60 mM of pH ranging between 7.3- 7.7, EDTA of concentration ranging between 20-30mM, Sarcosyl of concentration ranging between 0.3 - 0.8%, beta-mercaptoethanol of concentration ranging between 0.1-0.3 M.

In still another embodiment of the present disclosure, wherein the method maintains viability of mycobacteria and certain gram positive bacteria

In still another embodiment of the present disclosure, wherein homogenizing is required for time duration ranging between 20-120 seconds.

In still another embodiment of the present disclosure wherein Guanidinium hydrochloride of solution 1 lyses eukaryotic and Gram negative cells, denatures proteins, liquefies sample, and inactivates endogenous enzymes.

In still another embodiment of the present disclosure, wherein the processing is completed in a total time duration ranging between 1- 2 hours.

In still another embodiment of the present disclosure, wherein said method yields inhibitor-free mycobacterial DNA for PCR based diagnostics.

In still another embodiment of the present disclosure, wherein Universal Sample Processing (USP) solution for processing culture and smear samples comprises Guanidinium Hydrochloride (GuHCl) of concentration of about 4M, Tris-Cl of concentration ranging between 40-60 mM of pH ranging between 7.3- 7.7, EDTA of concentration ranging between 20-30mM, Sarcosyl of concentration ranging between 0.3 - 0.8%, beta-mercaptoethanol of concentration of about 0.1M.

In still another embodiment of the present disclosure, wherein said Universal Sample Processing (USP) solution for processing culture, smear, and PCR samples comprises Guanidinium Hydrochloride (GuHCl) of concentration of about 5M, Tris-Cl of concentration ranging between 40-60 mM of pH ranging between 7.3- 7.7, EDTA of concentration ranging between 20-30mM, Sarcosyl of concentration ranging between 0.3 - 0.8%, beta-mercaptoethanol of concentration ranging between 0.1-0.2 M.

In still another embodiment of the present disclosure wherein said Universal Sample Processing (USP) solution for processing smear, and PCR samples comprises Guanidinium Hydrochloride (GuHCl) of concentration of about 6M, Tris-Cl of concentration ranging between 40-60 mM of pH ranging between 7.3- 7.7, EDTA of concentration ranging between 20-30mM, Sarcosyl of concentration ranging between 0.3 - 0.8%, beta-mercaptoethanol of concentration of about 0.2 M.

In still another embodiment of the present disclosure, wherein the composition comprises solution 1 consisting of Universal Sample Processing (USP) solution, Solution 2 consisting of Sodium phosphate of concentration ranging between 65 to 70 mM of pH ranging between 6.7 to 6.8, Solution 3 consisting of Tween 80 of concentration ranging between 0.03 to 0.08%, and optionally, Solution A comprising Chelex 100 suspension of concentration ranging between 8-12%, and/or Solution B consisting of Triton X-100 of concentration ranging between 0.02-0.04%, and/or Tween 20 of concentration ranging between 0.2-0.4%

In still another embodiment of the present disclosure, wherein the use of Solution A, Solution B, and Solution C can be restricted to isolating DNA from low bacillary load samples only.

In still another embodiment of the present disclosure, wherein Guanidinium Hydrochloride (GuHCl), Sarcosyl, and beta-mercaptoethanol of USP act in a synergistic manner to perform optimal processing of all kinds of clinical samples.

In still another embodiment of the present disclosure, wherein presence of beta-mercaptoethanol and Sarcosyl is suitable for optimal processing of mucoid sputum samples.

In still another embodiment of the present disclosure, wherein presence of GuHCl is absolutely necessary along with beta-mercaptoethanol and Sarcosyl for optimal processing of mucopurulent sputum samples.

In still another embodiment of the present disclosure, wherein GuHCl acts as the principal inhibitor removal component in case of specimens containing blood, by denaturing hemoglobin and removing it from the specimen.

In still another embodiment of the present disclosure, wherein GuHCl acts as the principal decontaminating agent of clinical specimens.

In still another embodiment of the present disclosure, wherein PCR-amplifiable mycobacterial DNA can be obtained through simple lysis by boiling in presence of Solution 3 or by adding 0.03-0.1 % Triton X 100 without using Solution A, B and C in case of high bacillary load and/or lesser amount of junk containing samples.

In still another embodiment of the present disclosure, wherein the processed sample is free of contaminating organisms, proteins, enzymes, and interfering substances.

In still another embodiment of the present disclosure, wherein said method under smear microscopy can detect 300-400 bacilli/ml of the sample. The sensitivity (limit of detection) can be further increased by using more than the prescribed 10% of the processed specimen for smear microscopy.

In still another embodiment of the present disclosure, wherein said method has about 30 folds enhancement in sensitivity over the conventional direct smear microscopy method. In still another embodiment of the present disclosure, wherein said method in a smear shows sensitivity ranging between 97-99%.

In still another embodiment of the present disclosure, wherein said method in a smear shows specificity ranging between 83-92%.

In still another embodiment of the present disclosure, wherein said method shows enhancement in sensitivity by about 18% over CDC method of smear microscopy.

In still another embodiment of the present disclosure, wherein said method shows enhancement in sensitivity by about 30% over direct smear method.

In still another embodiment of the present disclosure wherein said method in smears shows positive predictive value ranging between 80-96%.

In still another embodiment of the present disclosure, wherein said method in smears shows negative predictive value ranging between 91 -99 %.

In still another embodiment of the present disclosure wherein said method in smears shows diagnostic accuracy of about 91%.

In still another embodiment of the present disclosure, wherein said method reduces counterstaining background enabling better viewing of the slides, enhances the gradation of slides, and reduces time and labour for reading a slide.

In still another embodiment of the present disclosure, wherein said method carries out decontamination of samples more efficiently as compared to the CDC method.

In still another embodiment of the present disclosure wherein said method in a culture shows twice the viability of the microbes as compared to CDC method.

In still another embodiment of the present disclosure, wherein said method in a culture leads to a lag of about 1 week in appearance of the microbial colonies as compared to untreated microbes.

In still another embodiment of the present disclosure wherein said method in a culture shows sensitivity of about 50%.

In still another embodiment of the present disclosure wherein said method is more suitable in than for CDC method in tropical areas.

In still another embodiment of the present disclosure, wherein said method in culture runs at a neutral pH.

In still another embodiment of the present disclosure, wherein said method in PCR shows no inhibition of PCR assay with any type of specimen tested.

The invention relates to a PCR using a set of primers namely, devRf3, & devRr3 of gene devR of microbe *Mycobacterium tuberculosis.*

In still another embodiment of the present disclosure, wherein the primers devRf2, and devRr2 amplify a 308bp fragment of gene *devR* of microbe *Mycobacterium tuberculosis.*

The present invention, using the primers devRf3, and devRr3 amplifies a 164 bp fragment of gene *devR* of microbe *Mycobacterium tuberculosis.* In still another embodiment of the present disclosure, wherein said method in PCR using primers devRf2 and devRr2 shows 2-4 folds increase in sensitivity as compared to devRf and devRr.

The present invention, using said method in PCR using primers devRf3 and devRr3 shows at least 10 folds increase in sensitivity as compared to primers devRf and devRr.

In still another embodiment of the present disclosure wherein samples can be obtained from sources comprising all types of sputum and other body fluids comprising FNAC, pus, pleural fluid, pericardial fluid, joint aspirate, peritoneal fluids, cerebrospinal fluids, endometrial aspirate, synovial fluid, gastric aspirate, endotracheal aspirate, urine, transtracheal aspirate, bronchoalveolar lavage, laryngeal swab and nasopharyngeal swab; body tissues comprising blood, pleural tissue, bone marrow and biopsy; solid organs comprising lymph node, bone, skin, and bovine samples comprising lymph gland, milk, and blood.

In still another embodiment of the present disclosure, wherein samples stored at about - 20°C for upto 2 months can be processed for PCR, smear-microscopy and culture.

In still another embodiment of the present invention, wherein said method in PCR can be used for both DNA, and RNA.

In still another embodiment of the present disclosure wherein said composition shows mucolytic, decontaminating, protein denaturant, chaotropic, liquefying, tissue softening/digesting, and mycobacteria-releasing action.

In still another embodiment of the present disclosure, wherein said method in smear enables more bacilli to be smeared on the slide thereby increasing the sensitivity and the efficiency. In still another embodiment of the present disclosure, wherein said method in smear generally converts slides that are graded as 1+ or scanty by the direct method to 2+/3+ or 2+/1+ respectively.

In still another embodiment of the present disclosure, wherein samples lacking purulence or containing nasopharyngeal discharge or saliva can be processed.

In still another embodiment of the present disclosure, wherein said method yields high quality smears from tissue biopsy samples suitable suitable for very sensitive AFB smear microscopy.

In still another embodiment of the present disclosure wherein the said method shows no adverse effect on the acid-fast properties, viability, and integrity of the *Mycobacterium tuberculosis.*

In still another embodiment of the present disclosure, wherein said method is suitable for diagnosis of pulmonary as well as extrapulmonary tuberculosis with equal efficacy.

In still another embodiment of the present disclosure, wherein said method is compatible with culturing mycobacterium from clinical specimens in both solid and liquid media.

In still another embodiment of the present disclosure, wherein the said method can detect samples as positive which have been detected negative by direct and CDC methods of smear microscopy.

In still another embodiment of the present disclosure, wherein a Kit useful in processing clinical samples for simple, rapid, safe, sensitive, and accurate diagnosis of microbial disease conditions, said kit comprising solution 1 consisting of Universal Sample Processing (USP) solution, Solution 2 consisting of Sodium phosphate of concentration ranging between 65 to 70 mM of pH ranging between 6.7 to 6.8 (optional, can be replaced with sterile water), Solution 3 consisting of Tween 80 of concentration ranging between 0.03 to 0.08%, Solution A comprising Chelex 100 suspension of concentration ranging between 8-12%, Solution B consisting of Triton X-100 of concentration ranging between 0.02-0.04%, and Solution C consisting of Tween 20 of concentration ranging between 0.2-0.4%, optionally two sets of primers with devRf2 and devRr2 of SEQ ID No. 1 and SEQ ID No. 2 respectively, and primers devRf3, and devRr3 of SEQ ID No. 3, and SEQ ID No. 4 respectively.

In still another embodiment of the present disclosure, wherein said kit is useful in processing clinical samples for detecting bacterial infections.

In still another embodiment of the present disclosure, wherein said kit is useful in processing clinical samples for detecting tuberculosis.

In still another embodiment of the present disclosure, wherein Universal Sample Processing (USP) solution comprises Guanidinium Hydrochloride (GuHCl) of concentration ranging between 3-6M, Tris-Cl of concentration ranging between 40-60 mM of pH ranging between 7.3- 7.7, EDTA of concentration ranging between 20-30mM, Sarcosyl of concentration ranging between 0.3 - 0.8%, beta-mercaptoethanol of concentration ranging between 0.1-0.3 M.

In still another embodiment of the present disclosure, wherein Universal Sample Processing (USP) solution for processing culture and smear samples comprises Guanidinium Hydrochloride (GuHCl) of concentration of about 4M, Tris-Cl of concentration ranging between 40-60 mM of pH ranging between 7.3- 7.7, EDTA of concentration ranging between 20-30mM, Sarcosyl of concentration ranging between 0.3 - 0.8%, beta-mercaptoethanol of concentration of about 0.1M.

In still another embodiment of the present disclosure, wherein said Universal Sample Processing (USP) solution for processing culture, smear, and PCR samples comprises Guanidinium Hydrochloride (GuHCl) of concentration of about 5M, Tris-Cl of concentration ranging between 40-60 mM of pH ranging between 7.3- 7.7, EDTA of concentration ranging between 20-30 mM, Sarcosyl of concentration ranging between 0.3 - 0.8%, beta-mercaptoethanol of concentration ranging between 0.1-0.2 M.

In still another embodiment of the present invention, wherein said Universal Sample Processing (USP) solution for processing smear, and PCR samples comprises Guanidinium Hydrochloride (GuHCl) of concentration of about 6M, Tris-Cl of concentration ranging between 40-60 mM of pH ranging between 7.3- 7.7, EDTA of concentration ranging between 20-30mM, Sarcosyl of concentration ranging between 0.3 - 0.8%, beta-mercaptoethanol of concentration of about 0.2 M.

In still another embodiment of the present disclosure, wherein the composition comprises solution 1 consisting of Universal Sample Processing (USP) solution, Solution 2 consisting of Sodium phosphate of concentration ranging between 65 to 70 mM of pH ranging between 6.7 to 6.8 (optional, can be replaced with sterile water), Solution 3 consisting of Tween 80 of concentration ranging between 0.03 to 0.08%, and optionally, Solution A comprising Chelex 100 suspension of concentration ranging between 8-12%, and/or Solution B consisting of Triton X-100 of concentration ranging between 0.02-0.04%, and/or Tween 20 of concentration ranging between 0.2-0.4%.

In still another embodiment of the present disclosure, wherein a set of primers devRf2 and devRr2 of SEQ ID No. 1 and SEQ ID No. 2 respectively.

The invention, relates to a set of primers devRf3, and devRr3 of SEQ ID No. 3, and SEQ ID No. 4 respectively.

In still another embodiment of the present invention, wherein a method of using primers of SEQ ID No. 3 and 4 of gene *devR* for screening patients of tuberculosis said method comprising steps of conducting Polymerase Chain Reaction (PCR) using *M. tuberculosis* DNA or RNA obtained from the processed sample of the subject, identifying the subjects suffering from tuberculosis.,

In still another embodiment of the present disclosure, wherein a processed clinical sample is obtained by processing a clinical specimen using method as described herewith.

In still another embodiment of the present disclosure, wherein samples can be obtained from sources comprising all types of sputum and other body fluids comprising FNAC, pus, pleural fluid, pericardial fluid, joint aspirate, peritoneal fluids, cerebrospinal fluids, endometrial aspirate, synovial fluid, gastric aspirate, endotracheal aspirate, urine, transtracheal aspirate, bronchoalveolar lavage, laryngeal swab and nasopharyngeal swab; body tissues comprising blood, pleural tissue, bone marrow and biopsy; solid organs comprising lymph node, bone, skin, and bovine samples comprising lymph gland, milk, and blood.

In still another embodiment of the present disclosure, wherein The method comprises of the use of a novel Universal Sample Processing (USP) solution. Briefly, the USP method comprises of homogenization, liquefaction and decontamination steps carried out on the clinical specimen by treatment with USP solution, concentration of mycobacteria by centrifugation and washing steps. Guanidinium hydrochloride comprises the active component of USP together with a reducing agent and a detergent in a buffered solution. This chaotropic agent Guanidinium hydrochloride effectively lyses eukaryotic cells, most bacteria excluding mycobacteria, denatures proteins, including mucin and thereby liquefying sputum, and inactivates endogenous enzymes including DNases and RNases. Proteins, debris and other contaminants are removed in subsequent rapid washing steps. The unique cell wall properties of mycobacteria render it selectively resistant to the action of chaotropic agent and the resultant bacteria are suitable for use in smear microscopy, culture, PCR, RNA isolation and procedures utilizing DNA and RNA isolated thereupon. The entire sample processing procedure can be completed in 1 ½ - 2 hours. The procedure does not involve steps of phenol:chloroform extraction, protease digestion, or precipitation steps, making the procedure ideal for processing many samples at a time. The method is applicable on all kinds of body fluids, blood, tissue biopsies, biopsies from vertebra and other bone joints and CSF. It is also suitable for use in processing tissue biopsies and milk of bovine origin. For body fluids other than sputum, concentration by centrifugation followed by USP solution and water wash is adequate for smear microscopy, culture, nucleic acid isolation and amplification. Tissue specimens require homogenization in USP solution prior to proceeding for the processing in the normal way.

The processed sample is directly used for smear microscopy, culture on solid media (such as Lowenstein Jensen (LJ) slants or Middlebrook 7H10 agar medium) or liquid medium (such as Middlebrook 7H9 medium used in MGIT system) and DNA and RNA extraction for amplification of mycobacterial DNA and RNA.

The duration of the methodology is as follows: 1 ½ -2 hours for sample processing, ½-1 hour for smear microscopy and culture and 4 hours for nucleic acid amplification.

[The USP Method described here, is expected to have extremely great utility in the rapid diagnosis of tuberculosis owing to its high sensitivity and applicability to all types of clinical material. This method will enable correct identifying of a sizeable population of infected individuals who escape detection by the less sensitive direct smear method and therefore this method has utility worldwide. The methodology described here is also compatible with culture and PCR/RT-PCR tests. To the best of our knowledge, no methodology has been described or is available which employs a single technology for smear microscopy, culture and PCR/RT-PCR on all types of clinical material for laboratory diagnosis of tuberculosis.

Thus it is clear that the method described here will find use in any laboratory that requires highly sensitive smear microscopy of acid-fast bacilli, effective decontamination of clinical samples for culture and the isolation of mycobacterial and *M. tuberculosis* DNA for PCR amplification, from clinical specimens. Therefore it has potential for inclusion in TB/mycobacterial diagnostic kits. The claimed technology can be used in a variety of clinical settings and laboratories. It can be used for smear microscopy in laboratories having minimal equipment (a light microscope and an ambient temperature centrifuge that can go up to a speed of 3000 rpm). It can be used for smear microscopy and culture in laboratories having additional facilities for mycobacterial culture. Finally PCR and RT-PCR test can be applied in sophisticated laboratories.]

The disclosure relates to a universal sample processing and diagnostic methodology to be used on any type of clinical specimen for the laboratory diagnosis of tuberculosis in a variety of clinical settings. It is rapid, inexpensive and easy and is compatible with smear microscopy, culture, DNA-RNA isolation and PCR/RT-PCR. It serves the quadruple purpose of (i) providing an extremely sensitive procedure for smear microscopy for the detection of *M.tuberculosis,* (ii) preparing the sample for culture on solid or liquid laboratory media, (iii) isolating DNA template free of PCR inhibitors and (iv) providing sensitive and specific diagnostic assays based on PCR. The methods have been evaluated on clinical specimens. USP smear microscopy showed a sensitivity of 97-99 % in comparison to 68.6 % by direct smear and 80 % by the CDC method. Using culture as gold standard, the 'short target' PCR assay yielded a sensitivity of 98.5 % vs the 'long target' PCR assay that showed a sensitivity of 73.2 %. In terms of usefulness for culture, the USP method was not significantly different from the conventional methods method; 50.1 % and 53.6 % positivity was noted for the USP and the conventional methods of culturing, respectively. Thus it is clear that the method described here will find use in any laboratory that handles *M. tuberculosis* and MOTT bacilli containing specimens and requires highly sensitive smear microscopy of acid-fast bacilli, effective decontamination of clinical samples for culture and the isolation of mycobacterial and *M. tuberculosis* DNA for PCR amplification, from clinical specimens. Therefore it has potential for inclusion in TB/mycobacterial diagnostic kits.

### Brief description of the Tables:

- **Table 1.**: Upgradation in smear status by USP smear microscopy over direct and CDC smear methods.
- **Table 2.**: Upgradation in smear status by USP smear microscopy over direct smear Method.
- **Table 3.**: Effect of USP solution on the viability of *M.tuberculosis.*
- **Table 4.**: Comparative sensitivity of *devR* PCR assays using different primer pairs and *M.tuberculosis* DNA.

### Brief description of the accompanying drawings

- **Fig. 1**: shows effect of variation in composition of USP solution on the processing of sputum specimens.
- **Fig. 2**: shows effect of variation in composition USP solution on smear microscopy of sputum specimens.
- **Fig. 3a**: shows pellets obtained for PCR after processing of sputum specimen using USP solution of varying composition.
- **Fig. 3b**: shows IS6110 PCR amplification profile *of M. tuberculosis* DNA isolated from the sputum specimen processed by USP solution of varying composition.
- **Fig. 4.**: Effect of variation in composition of USP solution on the culture of mycobacteria in sputum.
- **Fig. 5a**: effect of USP solution on sputa of different physical characteristics.
- **Fig. 5b**: shows effect of USP solution on a representative tissue biopsy specimen.
- **Fig. 6**: shows comparison of the smear prepared from the same sputum sample processed by USP, direct smear and CDC methods.
- **Fig. 7**: shows Limit of detection of acid-fast bacilli by the USP method of smear microscopy.
- **Fig. 8**: shows conversion of negative samples to positive by USP method of smear microscopy.
- **Fig. 9**: shows enhancement in smear status by USP method of smear microscopy as
- compared: to the direct and CDC methods of smear microscopy.
- **Fig. 10**: shows effect of USP solution treatment on the viability *of M. tuberculosis.*
- **Fig. 11**: shows primers for amplification of *devR* gene.
- **Fig. 12**: shows PCR amplification products obtained with DNA extracted from different mycobacteria using devRf3 and devRr3 primers.
- **Fig. 13**: shows PCR amplification products obtained with DNA extracted from different mycobacteria using devRf2 and devRr2 primers.
- **Fig. 14**: shows PCR amplification profiles of serial dilutions of *M.tuberculosis* DNA using three different primer pairs targeting *devR* gene.
- **Fig. 15**: shows comparison of PCR-amplifiable *M. tuberculosis* DNA isolation from sputum by lysis using solution 3, solution 3 with 0.01% Triton X-100 and lysis reagents (Solution A, B and C).
- **Fig. 16**: shows smear microscopy of USP-treated Mycobacteria other than *M. tuberculosis* (MOTT).
- **Fig. 17**: shows culture of MOTT bacilli after USP treatment.
- **Fig. 18**: shows amplification of DNA isolated from USP-treated MOTT bacilli.
- **Fig. 19**: shows RT-PCR reaction with *M. tuberculosis* RNA isolated from sputum.
- **Fig. 20**: shows Smear microscopy of USP-treated Gram-positive and Gram-negative bacteria.

### Brief description of the data sets:

- **Data set 1.**: Evaluation of USP smear vs. Direct smear methods.
- **Data set 2.**: Evaluation of USP method vs. CDC method of smear microscopy
- **Data set 3.**: Sensitivity and specificity of PCR using 'long target' and 'short target' *devR* PCR and comparison with IS6110 based PCR assay.
- **Data set 4.**: Smear microscopy and culture status of extrapulmonary specimens processed by the USP method.
- **Data set 5**.: Sensitivity and specificity values (%) for *devR-* short target PCR for the extrapulmonary samples.
- **Data set 6.**: Sensitivity and specificity values (%) for *IS6110* PCR for the extrapulmonary samples.

### Detailed description of the disclosure:

A multipurpose method has been developed for laboratory diagnosis of tuberculosis by smear microscopy, culture and PCR. The method is eminently suitable for the preparation of smears for highly sensitive microscopy. It effectively decontaminates samples making them suitable for culture and yields good quality inhibitor-free mycobacterial DNA for use in PCR- based diagnostic assays. It also enables the isolation of good quality mycobacterial RNA from clinical specimens employing common RNA isolation procedures.

The method for processing of sputum samples for smear microscopy, culture and PCR is as follows:

### Reagents:

**(i)** Solution 1: Universal sample processing Solution (USP): 4-6 M Guanidinium Hydrochloride (GuHCl), 50 mM Tris-Cl, pH 7.5, 25 mM EDTA, 0.5% solution of N-Lauroylsarcosine (henceforth referred to as sarcosyl), 0.1-0.2 M β-mercaptoethanol. (4M GuHCl and 0.1 M β- mercaptoethanol is preferred when only culture / culture and smear are to be performed; 5M GuHCl and 0.1-0.2 M β-mercaptoethanol is preferred in cases where culture is performed together with smear microscopy and PCR; 6M GuHCl and 0.2 M β- mercaptoethanol is preferred in cases where only smear microscopy, PCR and RT-PCR are done).
**(ii)** Solution 2: 68 mM sodium phosphate buffer, pH 6.8 (optional).
**(iii)** Sterile water (preferably double or triple distilled); (DEPC treated sterile water in case of RNA isolation.
**(iv)** Solution 3: Resuspension solution (0.05% Tween 80 in sterile water).

### For isolation of DNA from low bacillary load samples, the following additional reagents are needed:

**(i)** Solution A: 10% suspension of a resin, which is sodium salt of paired iminodiacetate ions coupled to styrene divinylbenzene matrix (henceforth ref erred to as Chelex 100).
**(ii)** Solution B: 0.03% Triton X-100.
**(iii)** Solution C: 0.3% Tween 20.

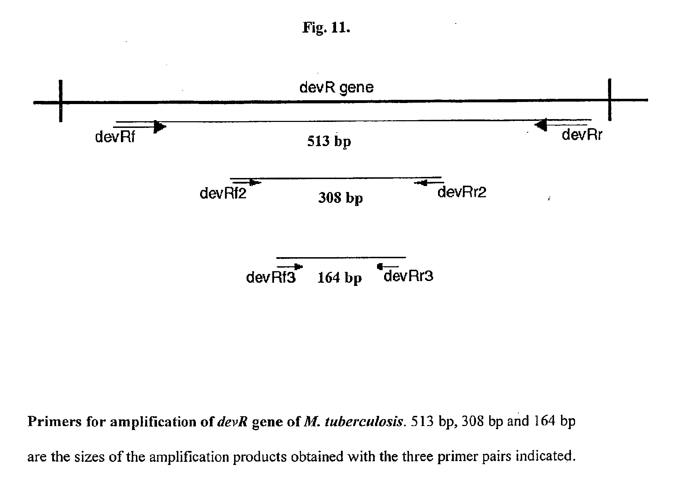

Note: For the isolation of DNA from smear positive samples and sterile fluids, solutions A, B and C are not required; amplifiable DNA may be isolated by directly heating the sample in Solution 3 at 90-100 °C for 30-40 minutes (or Solution 3 may be supplemented with Triton X-100 at a final concentration of 0.01 % (see Fig. 11).

### Methodology:

### Sample processing:

### Processing of sputum:

1) To the sputum sample add 1.5 to 2 volumes of Solution 1 in a 50 ml screw capped vial.
2) If possible, resuspend the sputum in USP solution using disposable plastic Pasteur pipette of 5 ml capacity.
3) Vortex well avoiding frothing (as much as possible) till the sputum is well homogenised (This is generally accomplished within 30-40 seconds). Complete homogenisation is necessary for optimal results (For highly purulent large volume and tenacious and hemoptyic sputum containing blood clots, incubation for 10 minutes at room temperature after vortexing may necessary).
4) Keep standing for 5-10 minutes at room temperature and add 10-15 ml of Solution 2 to the homogenate. This step aids in formation of a proper pellet. Mix gently and spin at 4000-5000 rpm at room temperature for 10-15 minutes in a laboratory centrifuge.
5) Discard the supernatant taking care that the pellet is not dislodged. If the pellet size does not reduce appreciably (to 1/10^{th} to 1/20^{th} of the original sputum volume), give another wash with about 2-5 ml of Solution 1.
6) Wash with sterile triple distilled water (while resuspending the pellet in water, it should be done only by vortexing. Optimal results may not be obtained if this is not followed.). The pellet is now ready for smear microscopy, culture, DNA/RNA isolation and PCR tests.

### Processing of uncoagulated blood (with EDTA):

1) Add an equal volume of Solution 1 to the blood sample and mix. If the blood is partially clotted, incubate with Solution 1 at 37°C for half an hour.
2) Pellet down the sample at medium (4000-5000 rpm, for sample volumes > 1.3 ml using 15 ml or 50 ml tubes) or high (12,000 -15,000 rpm, for sample volumes <1.3 ml using 1.5 ml tubes) speed.
3) If necessary wash the pellet once or twice with Solution 1 till a buff coloured pellet is obtained.
4) Wash the pellet with sterile triple distilled water. Resuspend the pellet in water by vortexing and not by pipetting. (Optimal results may not be obtained if this is not followed.).
5) The pellet is now ready for smear microscopy, culture DNA/RNA isolation and PCR tests.

### Processing of other body fluids (except sputum and stored pleural fluid samples):

1) For sample volumes > 1.3 ml: Pellet down the sample at medium speed (4000-5000 rpm) in a 15 ml or 50 ml screw capped vial.
2) For sample volumes <1.3 ml: Pellet down the sample at high speed (12,000-15,000 rpm) in a 1.5 ml polypropylene vial.
3) Note: In case of very thick pus samples add equal to 1^{1/2} volume of Solution 1 to the sample and mix by vortexing, and add some Solution 2 before pelleting. In case of bovine milk samples, remove the milk fat with a sterile cotton swab after centrifugation.
4) Wash the pellet with 1.5 - 2 times pellet volume of Solution lonce or twice followed by a wash with sterile triple distilled water. Resuspend the pellet in water by vortexing and not by pipetting. (Optimal results may not be obtained if this is not followed.). The pellet is now ready for smear microscopy, culture DNA/RNA isolation and PCR tests.

### Processing of pleural fluid:

If the pleural fluid is processed within 1 hour after collection, then processing can be carried out as described in "Processing of other body fluids." If the pleural effusion is stored for more than one hour the proteins separate out as a coagulum. Such a sample should be processed as follows:
1) Treat the coagulated protein (obtained by decanting the supernatant into another tube) separately with USP solution at 37°C or room temperature for 30 minutes till it dissolves.
2) Mix it with the supernatant and add some more Solution 1 and about 10ml Solution 2 and centrifuge at medium speed (4,000-5,000 rpm.) in a 15 ml or 50 ml screw capped vial.
3) If the pellet does not form properly add some more Solution 2 and pellet again.
4) To the pellet give a second wash with Solution 1 followed by water (10-15 ml) wash.
5) The pellet is now ready for smear microscopy, culture DNA/RNA isolation and PCR tests.
   Note: The pellet obtained from processing the liquid portion of the sample should be pooled with the processed pellet obtained from the coagulum. If the pleural fluid is collected in an EDTA vial, which prevents formation of coagulum, processing can be carried out as described in " processing of other body fluids."

### Processing of tissue biopsy (Fresh and paraffin-embedded):

1) Add Solution 1 to the vial containing the biopsy after removing the normal saline or the fluid in which it is contained, and transfer it to a sterile Petri dish after 15-20 minutes.) This aids to the softening of the tissue material, which is necessary for the subsequent processing.
2) After the biopsy material becomes soft, mince it as finely as possible with sterile scalpel. The mincing should be fine to get optimal results.
   (Note: With fine needle biopsy, mincing is not necessary)
3) Transfer the minced biopsy material into a mini bead-beater vial (1.5 ml capacity) containing at least half the volume of sterile 1 mm glass beads. The volume should not exceed 1 ml.
4) Beat in a mini bead beater (Mini-Beadbeater^{™}, Biospec Products, USA) for 30-60 seconds. Repeat bead beating once more for 30 seconds if the material is not homogenized properly.
5) Centrifuge the vial at 2000 rpm for two minutes and collect the homogenate into a fresh vial avoiding collection of any glass beads.
6) Centrifuge the homogenate at 12000-15000 rpm for 10 minutes at 25°C and discard the supernatant.
7) Wash the pellet once again with Solution 1 (1ml volume maximum) followed by a wash with sterile water.
8) The pellet is now ready for smear microscopy, culture DNA/RNA isolation and PCR tests.
   Note: For paraffin embedded tissue samples, deparaffinization with xylene is necessary before processing.

### Smear microscopy, culture and PCR technique:

To the pellet obtained after wash with sterile water, add 500 µl of Solution 3 and resuspend the pellet thoroughly. It may be noted that the pellet has a tendency to sediment along the wall of the tube as well as at the bottom after centrifugation. Care is to be taken that the pellet is completely resuspended to yield a homogeneous solution. This is important to obtain optimal results. Transfer it to a 1.5 ml polypropylene vial. The material is now ready for the three tests namely, smear microscopy, culture and PCR.

### A). Smear examination (10% of the re-suspension is used)

Smear 50 µl on a clean glass slide in a minimum area possible. If there is any particulate matter in the smear, care should be taken to spread it out evenly with the inoculation loop while smearing. Area of smearing preferably should not exceed 1 cm X 1 cm.

Simplified method of USP smear preparation: Add 2-3 volumes of USP solution to the sputum (specimen volume less than or equal to 2 ml) collected in McCartney bottles. Mix by hand for 30-60 second to homogenize well. Allow to stand for 5-10 minutes. For highly tenacious and purulent sputum specimens, add at least 3 volumes of Solution 1, hand mix and incubate at 37°C for 20-30 min. Fill the bottle with sterile water upto 2/3^{rd} level and mix by inversion. Centrifuge at 3,000g for 20 minutes in an ambient temperature clinical centrifuge. Decant supernatant carefully taking care that the pellet is not dislodged/lost. Re-suspend the pellet in 2-5 ml of Solution 1 and centrifuge as before. Decant the supernatant carefully and wash the pellet with ∼10 ml sterile water. Decant the supernatant carefully, add ∼10 drops of solution 2 with a dropper/Pasteur pipet to the pellet and re-suspend it thoroughly with the help of an inoculation loop. Take care to include in the final re-suspension the pellet deposited along the wall of the bottle during centrifugation. Pour 3-4 drops of the re-suspension on a clean slide and spread using a continuous rotational movement with an inoculation loop to produce a smear of about 20 mm by 10 mm. Allow the smear to air-dry for about 30 to 45 min, fix over flame, stain for AFB using ZN stain and examine slide under oil-immersion lens using a light microscope.

### B.) Culture (50% of the resuspension is used)

Inoculate 200 µl onto LJ media. Incubate at 37°C.

### C.) DNA isolation for PCR (40% of the resuspension is used)

For DNA isolation, pellet the 'Solution 3 resuspension' at 12,000 - 13,000 rpm in a microcentrifuge tube. To the pellet add approximately 5 times the volume of Solution A, and 1/10^{th} volume of each of Solution B and Solution C, as that of Solution A. Mix well, avoiding frothing. Heat at 90°C for 40 minutes. Centrifuge at room temperature at 12,000 rpm for 5-10 minutes. Perform PCR with supernatant. Be careful not to add any debris or resin from Solution A into the PCR reaction.

Note: If the pellet size is drastically reduced so that it is not visible at all or the pellet size is very minute or the sample is a high bacillary load sample, lysis may be carried out by heating the 'Solution 3 resuspension' directly or after adding Triton X-100 (at a final concentration of 0.01 %) at 90-100 °C for 30-40 minutes. The supernatant may be used directly for PCR. The addition of the lysis reagents (namely, solutions A, B and C) may be avoided in such cases; but for best results, it is recommended that the lysis reagents (Solution A, B and C) be used for isolation of PCR-amplifiable DNA (See below for details). D). RNA isolation: The pellet obtained can be subjected to mycobacterial RNA isolation by any standard RNA isolation procedures or kits.

### PCR Technique

The PCR assay targets the *devR* gene (*Rv3133c*) of *M. tuberculosis* and is specific for the organisms of the *M. tuberculosis* complex [Singh *et al*., 1999]. In published studies from our laboratory, the primer pair devRf and devRr was used in the 'long target' PCR assay that amplified a 513 bp fragment from the *devR* gene [Singh *et al*., 1999; 2000]. In a multi-centric study under DBT-funded project entitled "Evaluation of PCR-based test for tuberculosis", [duration Feb 1997- Feb 2000, with financial support till 28.2.1999], the same PCR assay was used with coded sputum samples. The results of smear microscopy and culture were correlated with PCR results. Sensitivity was 100% for culture positive (14/14 culture positive) and 94% for smear positive samples (17/18 smear positive samples) [Ref: DBT Report on the analysis of the results of evaluation of different indigenously developed PCR assays for the diagnosis of tuberculosis. DBT, Govt. of India].

It was reasoned that amplification of a smaller region of the *devR* gene was likely to provide equivalent / better sensitivity with paucibacillary and smear negative samples. With this idea in mind two new primer pairs were designed to amplify shorter fragments of the *devR* gene. The 'short target' PCR assays were assessed first on purified *M. tuberculosis* DNA and subsequently validated on clinical specimens. The 'short target' primer pairs are, (i) devRf2, 5' TGGCAACGGCATTGAACTGT 3'and devRr2, 5' TAAGCAGGCCCAGTA GCGT 3' and (ii) devRf3, 5' ATCTGTTGTCCCGCATGCC 3' and devRr3, 5' GTCCAGC GCCCACATCTTT 3'.

### The following PCR parameters were used:

'Short target' assay: initial denaturation at 94°C for 10 minutes; 45 cycles of denaturation at 94°C for 1 minute, annealing at 52°C for 1 minute and extension at 72°C for 30 seconds; and a final extension at 72°C for 10 minutes.
'Long target' assay: Identical parameters as for the 'short target' assay except that annealing and extension were performed at 65 °C for 90 seconds.

IS6110 assay: Published primers suggested by Eisenach *et al.* (1990) were used with the PCR reaction parameters: initial denaturation at 94°C for 10 minutes followed by 45 cycles of 94°C for 1 min. and 60°C for 1min. 30 seconds, followed by a final extension at 72°C for 5 minutes.

### Reaction components:

| Cocktail Components | Stock concentration | Working concentration |
|---|---|---|
| 1.) PCR reaction buffer | 10 X | 1 X |
| 2.) MgCl₂ | 25 mM/50 mM | 1.5 mM |
| 3.) Primers | 20 µM* | 0.5 µM |
| 4.) dNTPs | 10 mM | 0.2 mM |
| 5.) Taq polymerase | 5U/µl | 1U |

| | | |
|---|---|---|
| *To be reconstituted in PCR-grade water to obtain 20 µM concentration. | | |

Total reaction volume is 40µl (30µl cocktail and 10µl template). Dispense 30µl cocktail into sterile 0.2 ml or 0.5 ml vials in a DNA-free hood. Add 10µl template in a designated area away from the PCR-setup area. Give the tubes a brief spin for 30 seconds and place them in the thermal cycler. After the completion of the reaction (parameters given above) electrophorese the amplification products in 1.5-2.3 % agarose gel in 0.5 × TBE buffer at 80 volts for 20-30 minutes. Visualize the product (load at least 35 µl) by ethidium bromide staining under UV light.

### 8. Examples.

**(A) Function of USP solution components:** To check for the function of each component of the USP solution, the USP solution was selectively depleted of the individual components and samples processed with the resulting solutions. USP solution consists of five components:
   1. Guanidinium hydrochloride (GuHCl)
   2. Sarcosyl
   3. β-mercaptoethanol.
   4. Tris-HCl
   5. EDTA

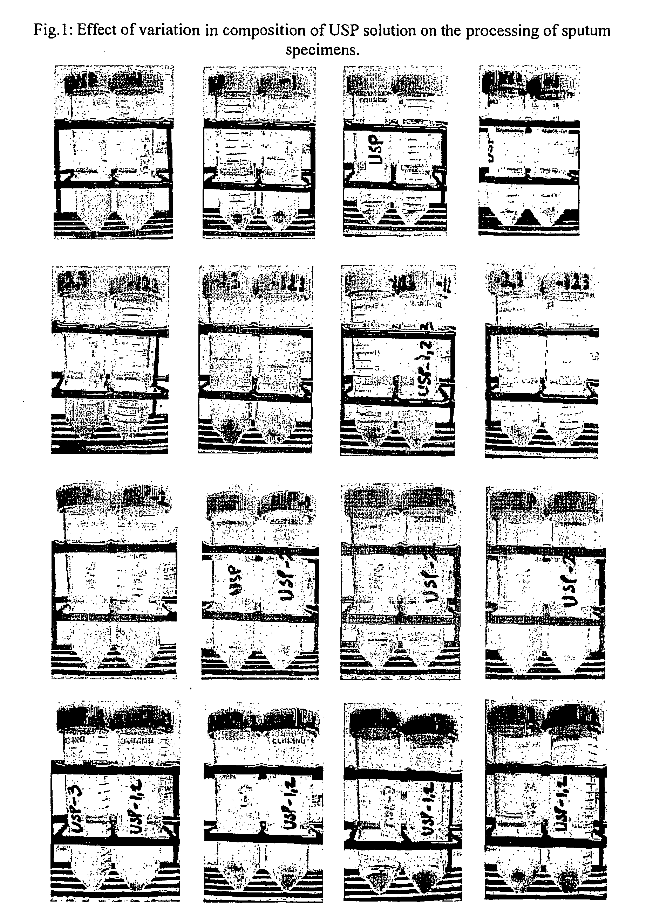

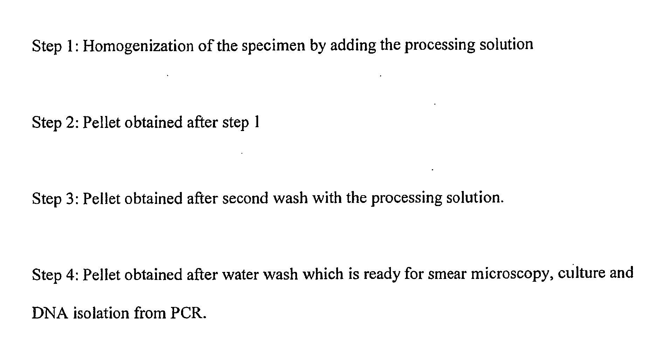

It was apparent that the mucolytic, decontaminating, protein denaturing, chaotropic, liquefying, tissue softening/digesting, mycobacteria-releasing actions which are the principal functions of USP solution are performed by the first three components, namely GuHCl, Sarcosyl and β-mercaptoethanol. To establish this, these three components were selectively depleted from the USP solution individually or in combination keeping the Tris-HCl and EDTA composition unchanged. The selectively depleted solutions were assessed for their effect on specimen processing, smear microscopy, PCR and culture (Fig. 1 to 4). A sputum specimen (obtained by pooling three to four AFB positive sputa (to obtain a large volume and heterogeneous composition in terms of contaminants), was homogenized by vortexing with 3 mm glass beads for 3-5 minutes. Aliquots of 5 ml each of the homogenized sputum were distributed for processing with the solutions of different compositions. USP solution was used as a control. The following solutions of varying compositions were prepared for sample processing:
1. USP solution containing all the components (called USP).
2. USP solution without GuHCl (called USP-1).
3. USP solution without Sarcosyl (called USP-2).
4. USP solution without β-mercaptoethanol (called USP-3)
5. USP solution without GuHCl and Sarcosyl (called USP-1,2)
6. USP solution without GuHCl and β-mercaptoethanol (called USP-1,3)
7. USP solution without GuHCl, Sarcosyl and β-mercaptoethanol (called USP-1,2,3)
8. USP solution without Sarcosyl and β-mercaptoethanol (called USP-2,3).

All the solutions were used for processing of the sputum specimen as described and smear microscopy, PCR and culture was performed. The experiment was repeated twice for samples with varying physical characteristics.

### The principal observations were:

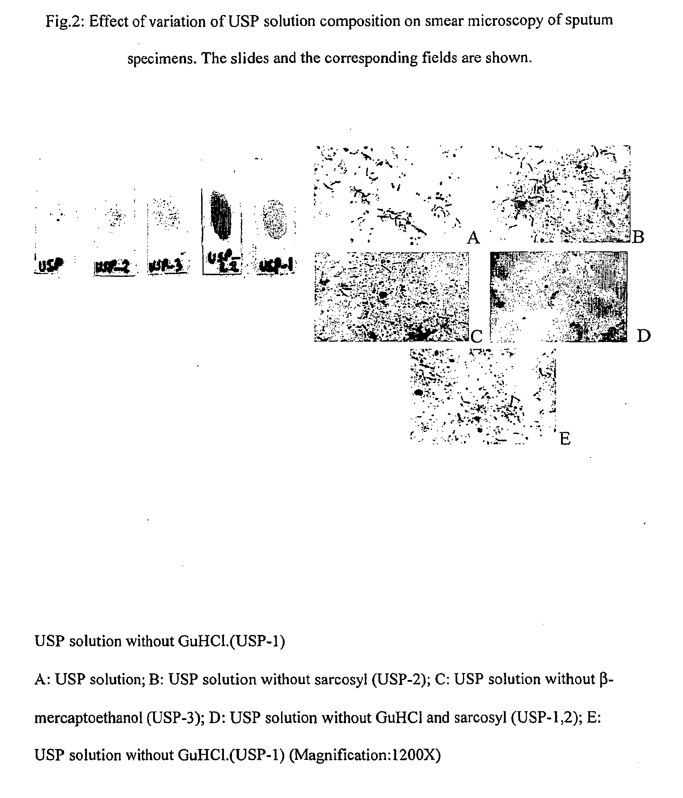

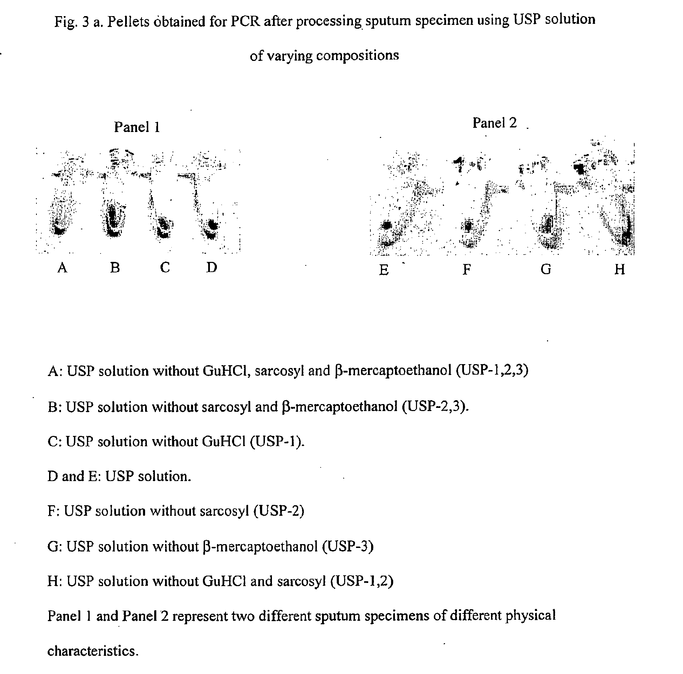

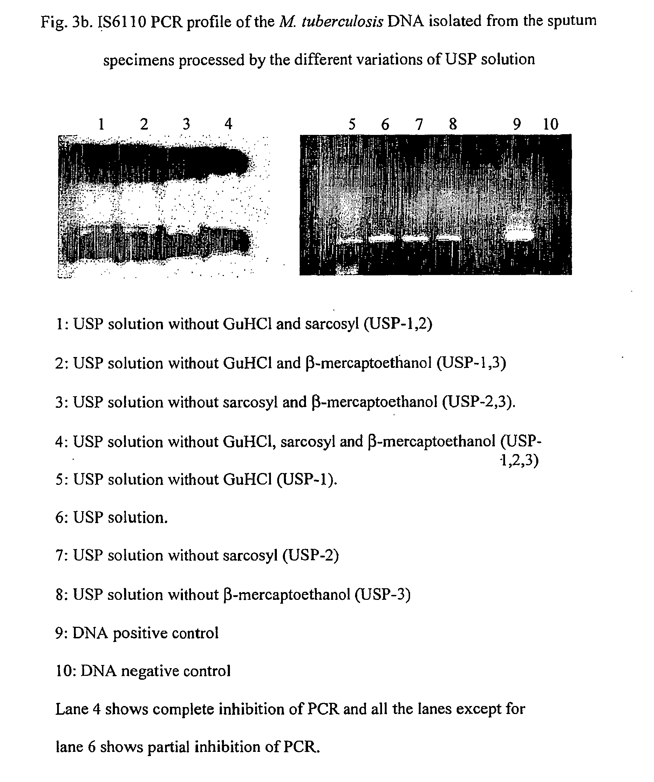

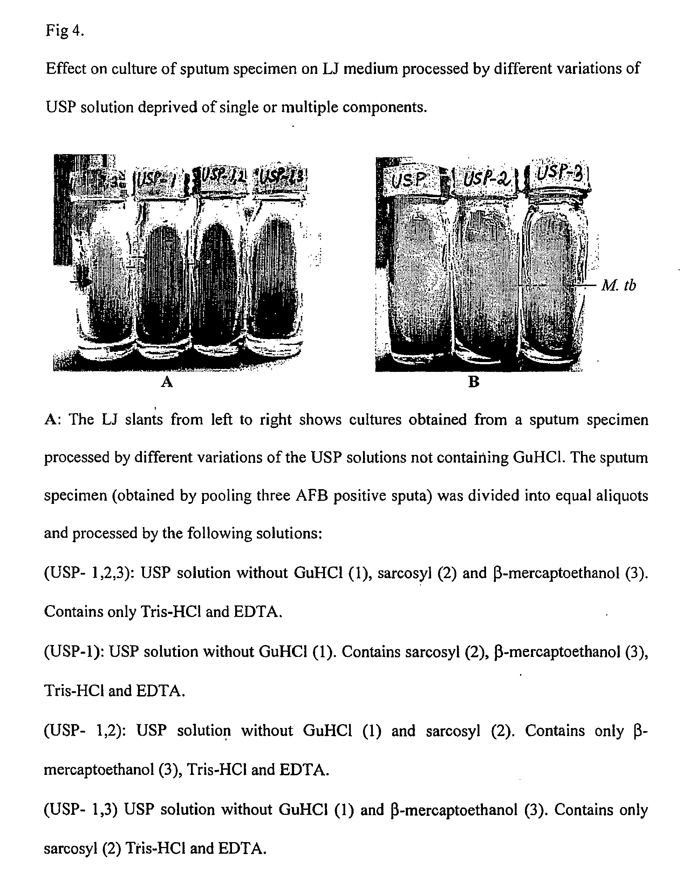

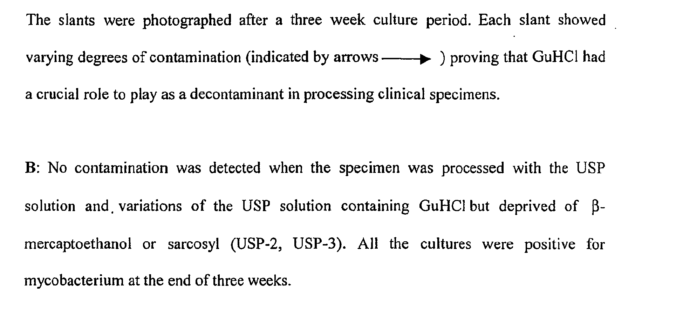

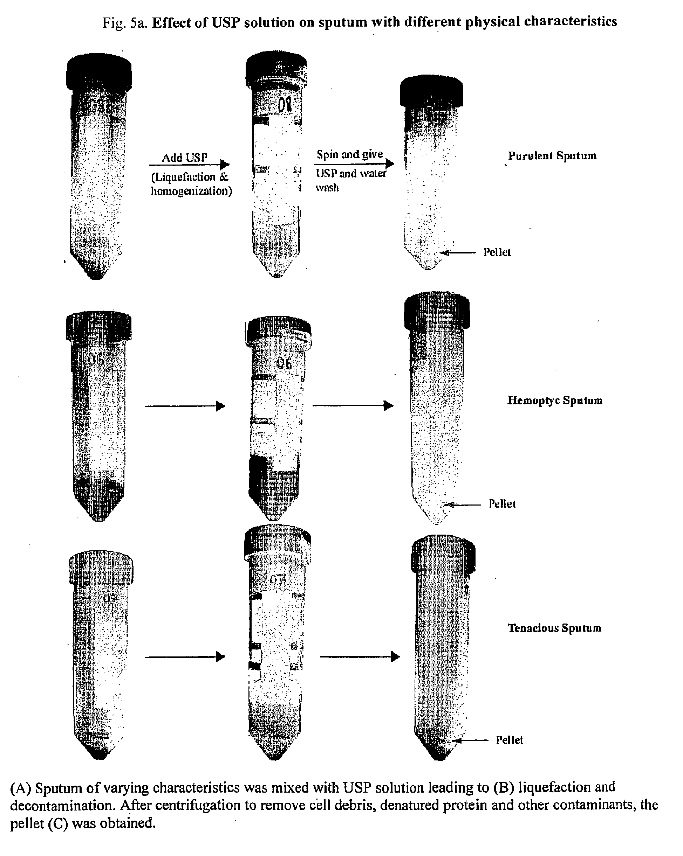

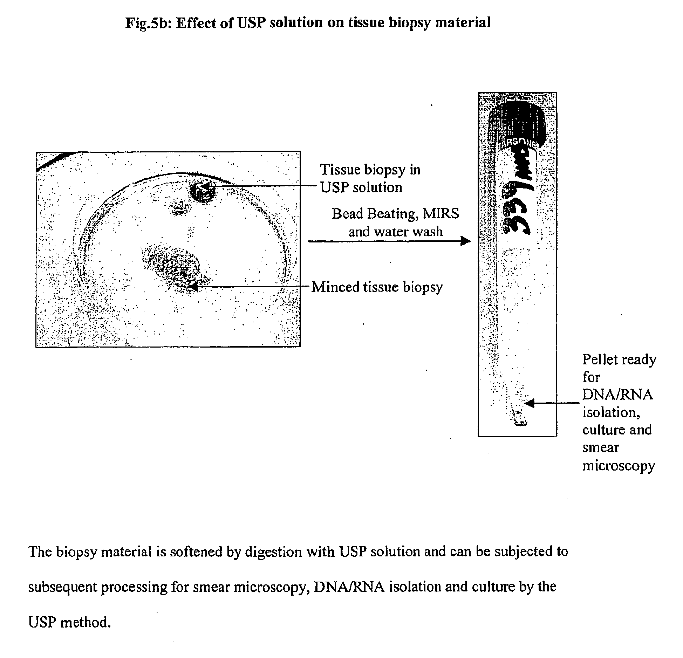

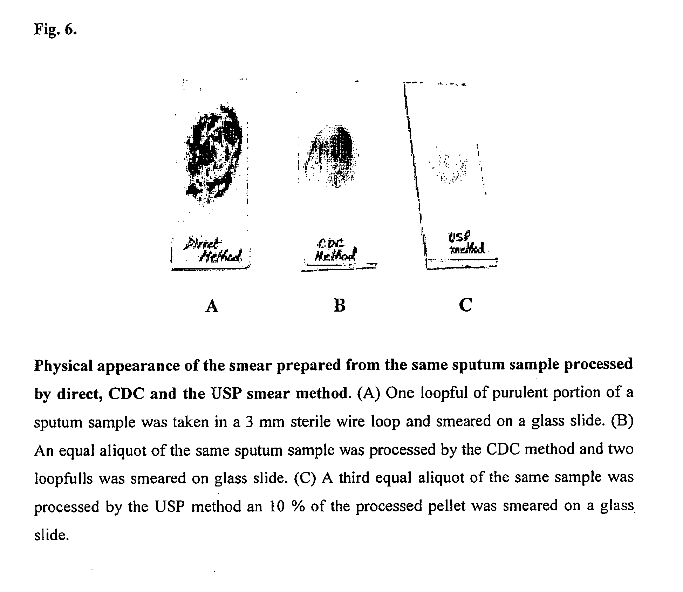

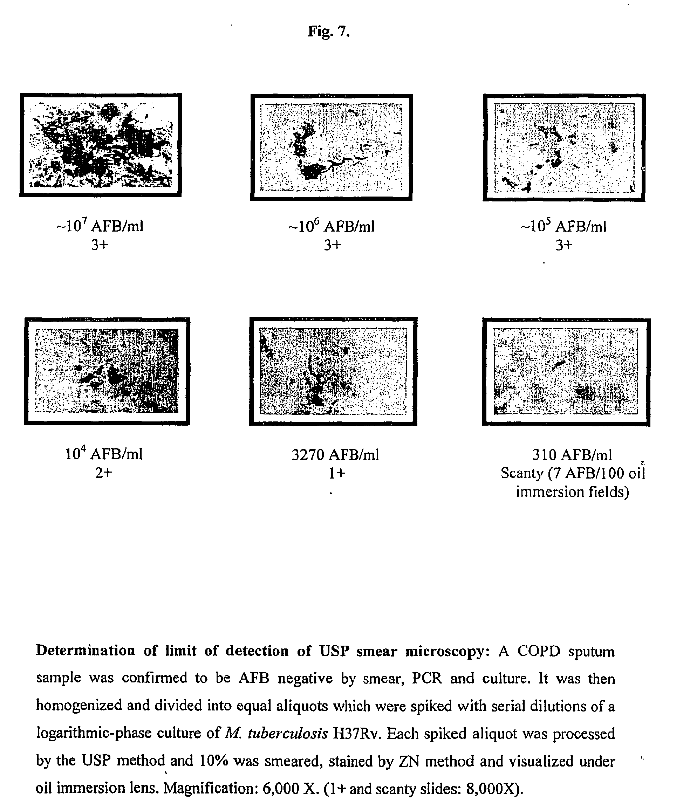

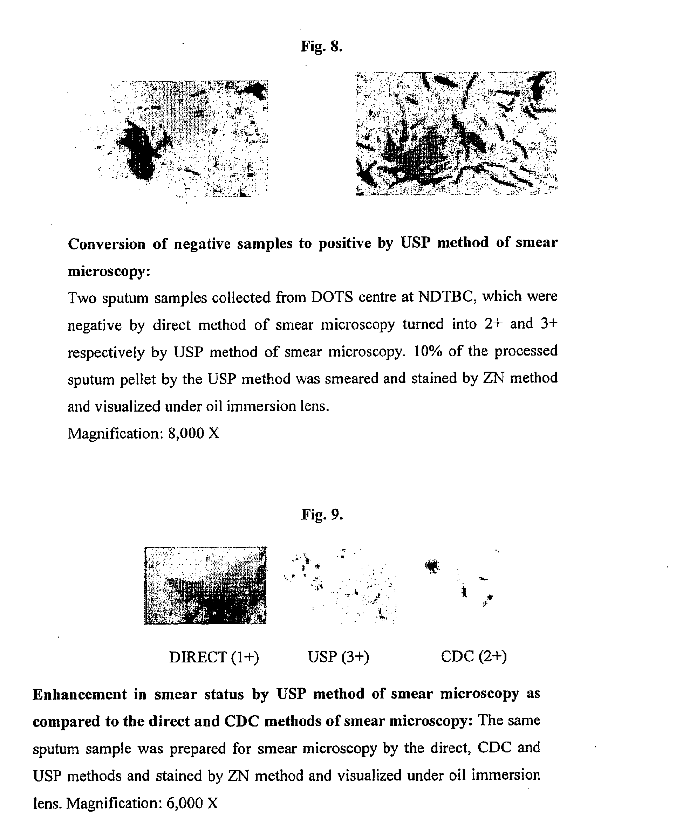

1. There was no reduction in pellet size when the sputum specimens were processed with USP solution lacking the three principal components (USP-1,2,3, Fig.1). Excessively thick smears with a lot of background were obtained when 10 % of the final processed pellet was smeared onto the slide, which was very difficult to read. Culture contamination came within two days of inoculation on LJ medium and PCR showed inhibition.
2. Whatever may be the variation of composition, the USP solution always fared the best in terms of junk removal and PCR inhibitor removal from the sample, producing the most clean pellet from the sputum specimen (Fig.1, Fig. 3).
3. For sputum specimens containing blood, GuHCl acted as the principal inhibitor removal component of the sample by denaturing the hemoglobin and removing it from the sample. For specimens with high purulence and blood, PCR inhibition was noted in the DNA preparations of samples processed with USP solution not containing GuHCl.
4. It was seen that in case of mucoid sputum samples, USP solution that contained only Sarcosyl and β-mercaptoethanol [i.e. without GuHCl (USP-1)], effectively processed the sample with commendable efficiency and better than USP containing only GuHCl (USP-2,3) as far as smear microscopy and PCR were concerned (Fig.1, 2 and 3). However, USP-1 failed to adequately process (in terms of efficiency of junk removal and inhibitor removal) sputum specimens of high purulent nature. In such cases USP solution containing only GuHCl (USP-2,3) or GuHCl with Sarcosyl (USP-3) fared better. So it was evident that the presence of GuHCl and Sarcosyl in USP was more suitable for purulent samples and the presence of β-mercaptoethanol and Sarcosyl was more suitable for mucoid samples. Though even for mucoid samples, the presence of GuHCl was necessary to remove PCR inhibition efficiently. But for mucopurulent samples the presence of GuHCl along with β-mercaptoethanol and/or Sarcosyl was absolutely necessary for optimal processing.
5. Whenever GuHCl was subtracted from the USP solution, the resulting cultures showed contamination when inoculated on LJ medium (Fig. 4A). Presence of GuHCl in the solution was necessary for proper decontamination of the specimens.
6. Depletion of any one of the principal components resulted in increased background in the smears when smears were prepared by using 10 % of the final processed pellet (Fig. 2), which interferes with ease in slide reading which is a major advantage of the USP method (see following sections).
7. Thus it was evident that the three principal components in the USP solution act in a synergistic manner to produce the optimal effect in processing the sample and the absence of any one of these components results in sub-optimal results. The versatility of the USP solution in processing specimens of a wide range of physical characteristics and biochemical nature with equal efficiency can be attributed to the synergistic effect of all the components of the USP solution. Tris-HCl helps in maintaining the neutral pH of the USP solution and thus there is no need for neutralization of the USP solution-treated specimen before culturing.
   EDTA acts as a chelator of positive ions, thereby inactivating DNase. GuHCl is a potent chaotrope thereby helping in denaturing all the host proteins in the specimen including mucin and hemoglobin thereby removing potential PCR inhibitors and counterstaining material from the clinical specimen. It kills Gram-negative bacteria present in the specimen thereby decontaminating it (see below). It also denatures DNases and RNases thereby preserving nucleic acids and making the procedure amenable to DNA and RNA isolation. Sarcosyl solubilizes lipids and disrupts cell membranes of eukaryotic cells and Gram negative bacteria and helps in decontaminating the sample and releasing mycobacteria from macrophages. β-mercaptoethanol, a reducing agent acts as a mucolytic component reducing the S-S bonds of mucin to -SH groups and releases mycobacteria trapped in the mucous. The tough cell wall characteristics of mycobacteria makes it totally inert to any adverse effect by the USP solution and thus the method is eminently suitable for processing samples as a prelude to Mycobacterial diagnostics by smear microscopy, culture, PCR and RT-PCR (for RNA).
   **(B) Rapid liquefying activity of USP.** Fig. 5a and Fig. 5b show representative sputum and tissue samples treated with USP. At the end of the process the sample is rid of contaminating organisms, proteins, enzymes and interfering substances and considerably reduced in volume.
   **(C) Smear microscopy.** Representative slide smears prepared by the USP, CDC and direct smear methods are shown in Fig. 6. The appreciable decrease in background staining material is noteworthy in the USP slide.
      **(i) Sensitivity of the USP method of smear microscopy.** *M.tuberculosis* H37Rv was grown up to logarithmic phase in Middlebrook 7H9 medium containing ADC and 0.05% Tween 80. The culture was serially diluted in duplicate sets as follows: 1:10, 1:100, 1:1000 and so on till 1:100000. 10% of each dilution was inoculated on LJ slants (solid medium) and 100% of each of the dilution the other set was spiked into 1 ml sputum aliquots from a patient suffering from chronic obstructive pulmonary disease (COPD) without any history or symptoms of TB. The spiked samples were processed by the USP method and 10% of each sample was smeared on a glass slide, stained for AFB and microscopically examined under oil immersion lens. A similar experiment was performed using COPD sputum samples spiked with *M. smegmatis,* a non-pathogenic fast growing mycobacterium. From both the experiments it was established that the USP method of smear microscopy could detect as positive, samples that contained 300-400 AFB/ml of the sample. The results were verified twice more. A representative experiment is shown in Fig. 7. These results clearly illustrate the superior sensitivity of the USP method of smear microscopy. A ∼30 fold enhancement in sensitivity over the conventional direct smear microscopy method was noted. The sensitivity of detection of the direct method its ∼10,000 bacilli/ml of sample (Kent and Kubica, 1995).
      **(ii) Evaluation of USP smear microscopy in a clinical setting.** The performance of USP smear microscopy was evaluated on a panel of 571 sputa collected from patients (n = 571) attending the DOTS centre at New Delhi Tuberculosis Centre, New Delhi; Sunderlal Jain Charitable Trust Hospital, Delhi and Tuberculosis Research Centre, Chennai for diagnosis of pulmonary tuberculosis. The patients chosen for this study were evaluated for any of the following clinical symptoms namely, fever, cough, expectoration of sputum, haemoptysis, pain, dyspnoea and other symptoms like loss in weight, night sweats and general weakness, X-ray chest, Mantoux status and any past history of tuberculosis. Any specimen collected from a subject already on ATT at the time of specimen collection was excluded from the study. Culture results on LJ media was used as a gold standard. All the specimens were coded and the study was blinded.
      **(iii)**The performance of USP method was evaluated with respect to both the direct smear and the NALC-NaOH concentration methods, the two most universally applied methods of smear microscopy. All 571 samples were processed for smear microscopy by the direct method and the USP method (Data set 1). Out of these, 325 samples (Data set 2) were also processed by the NALC-NaOH concentration method (developed at CDC, Atlanta and called CDC method). The USP and CDC methods were performed on what were considered to be approximately equal aliquots of the sputum samples. The direct smears were independently prepared and read at two different sites by different trained personnel. The smears prepared by the USP and CDC methods were read by at least two trained personnel at one laboratory. The improvements in USP smear method over the direct smear method and the CDC method are:
         a). Negative samples turn to positive in USP smear: The sensitivity of the direct smear method and the USP smear method were 68.6 % and 98.2 %, respectively, with a specificity of 92.6% and 91.4 % respectively on the panel of 571 sputum sample (Data set 1). All samples diagnosed as smear positive by the direct method were also positive by the USP and CDC methods of smear microscopy. Out of the 325 direct smear-negative samples, the USP method detected 97 additional samples as smear positive, which were positive by culture also, thereby recording an enhancement in sensitivity of 29.6 %. A representative example is shown in Fig. 8. Out of the 97 USP smear-positive samples, 14 were graded as scanty, 29 as 1+, 23 as 2+ and 31 as 3+.

The USP smear method was also simultaneously compared with CDC method of smear microscopy in 325 out of these 571 specimens (Data Set 2). 29 specimens, which were smear negative by the CDC method, were detected as positive by the USP method of smear microscopy all of which were also culture positive. Thus USP method also fared better than a popularly used concentration method of smear microscopy (CDC method) in terms of sensitivity of detection (97.1% and 80% sensitivity for USP and CDC smear microscopy, respectively).

### Data set 1

### Evaluation of USP smear vs. Direct smear methods

### Gold Standard: Culture (on LJ media), Sample strength, n = 571

| **Smear method and result** | **Culture** | |
|---|---|---|
| | Positive | Negative |
| **USP** | | |
| Any positive | 322 | 21 |
| Negative | 6 | 222 |
| Total | 328 | 243 |
| **Direct** | | |
| Any positive | 225 | 18 |
| Negative | 103 | 225 |
| Total | 328 | 243 |

### Measure of association at 95% confidence interval

| **USP smear microscopy (ZN)** | |
|---|---|
| Sensitivity: 98.2 % | (95.9 %-99.3 %) |
| Specificity: 91.4 % | (86.9 %-94.4 %) |
| PPV: 93. 9 % | (90.7 %-96.1 %) |
| NPV: 97.4 % | (94.1 %-98.9 %) |
| Diagnostic accuracy: 90.5 % | |

### Direct smear microscopy (ZN)

| | |
|---|---|
| Sensitivity: 68.6 % | (63.2 %-73.5 %) |
| Specificity: 92.6 % | (88.4 %-95.4 %) |
| PPV: 92.6 % | (88.4 %-95.4 %) |
| NPV: 68.3 % | (62.9 %-73.3 %) |
| Diagnostic accuracy: 78.3 % | |

### Data set 2

### Evaluation of USP method vs. CDC method of smear microscopy (ZN)

Gold standard: Culture (on L J media)
Sample strength, n = 325.

### Measure of association at 95% confidence interval.

| | Culture | |
|---|---|---|
| Smear method and result | Positive | Negative |
| **USP** | | |
| Any positive | 165 | 26 |
| Negative | 5 | 129 |
| Total | 170 | 155 |
| **CDC** | | |
| Any positive | 136 | 16 |
| Negative | 34 | 139 |
| Total | 170 | 155 |

### USP smear microscopy (ZN)

| | |
|---|---|
| Sensitivity: 97.1 % | (92.9 % - 98.9 %) |
| Specificity: 83.3 % | (76.2 % - 88.6 %) |
| PPV: 86.4 % | (80.5 % - 90.8 %) |
| NPV: 96.3 % | (91.1 % - 98.6 %) |
| Diagnostic accuracy: 90.5 % | |

### CDC smear microscopy (ZN)

| | |
|---|---|
| Sensitivity: 80.0 % | (73.0 % - 85.6 %) |
| Specificity: 89.67 % | (83.5 % - 93.8 %) |
| PPV: 89.47 % | (83.2 % - 93.7 %) |
| NPV: 80.35 % | (73.5 % - 85.8 %) |
| Diagnostic accuracy: 84.6 % | |

b). Upgradation in smear status: The USP method enhances the gradation of slides making them easier to read in a shorter period of time. Slides graded as scanty, 1+ or 2+ by the direct method of smear microscopy generally turned into 1+, 2+ or 3+ by the USP method. This trend was observed when the results of USP method of smear microscopy were compared with that of the direct method over the panel of 571 samples (Table 1). The upgradation in the smear status was also observed when the USP solution method was compared with the CDC method (Table 2). Representative slides are shown in Fig. 9. Thus the USP method also fared better than the CDC method in terms of the number of bacilli observable per field. This facilitated rapid and efficient reading of the slides ultimately resulting in saving of the technician's time and labour.

**Table 1: Upgradation in smear status by USP smear microscopy over direct smear method (Total sample strength = 571; samples positive by direct method, n = 243)**

| Direct Smear Examination (n=243) | | USP smear examination no. |
|---|---|---|
| Grade | Sample | |
| Scanty | 29 | 1+ (4) |
| | | 2+ (15) |
| | | 3+ (10) |
| 1+ | 97 | 2+ (40) |
| | | 3+ (57) |
| 2+ | 70 | 3+ (70) |
| 3+ | 47 | 3+ (47) |

| | | |
|---|---|---|
| (Values in parenthesis indicate sample strength). | | |

**Table 2: Upgradation in smear status by USP smear microscopy over CDC smear method (Total sample strength = 326; samples positive by CDC method, n = 152)**

| CDC Smear Examination (n=152) | | USP smear examination |
|---|---|---|
| Grade | Sample no. | |
| Scanty | 13 | 1+ (9) 2+ (4) |
| 1+ | 35 | 1+ (7) 2+ (20) 3+ (8) |
| 2+ | 37 | 3+ (26) 2+ (11) |
| 3+ | 67 | 3+ (67) |

| | | |
|---|---|---|
| (Values in parenthesis indicate sample strength) | | |

Thus the USP solution method of smearing showed a very high sensitivity in the range of 97-98 %, much better than the other two methods that were tested, with a specificity range of 83-91 %. USP smear microscopy showed a positivity of 97-98 % in culture positive samples vs. 80 % by CDC and 68.6 % by direct smear methods thereby recording an enhancement in sensitivity of ∼ 30 % and 18 % over the direct and CDC method of smear microscopy respectively. The increase in the sensitivity was significant as the sensitivity values of all the three methods were completely non-overlapping at a 95 % confidence interval. The USP smear method did not miss a single sample detected by either the direct smear method or the CDC method. The specificity of the USP smear microscopy method when compared to the direct smear method was 91.3 % and that when compared to the CDC method was 83.3 %. The two values were overlapping at a 95 % confidence interval.

### (Data Sets 1 and 2).

### (D) Culture.

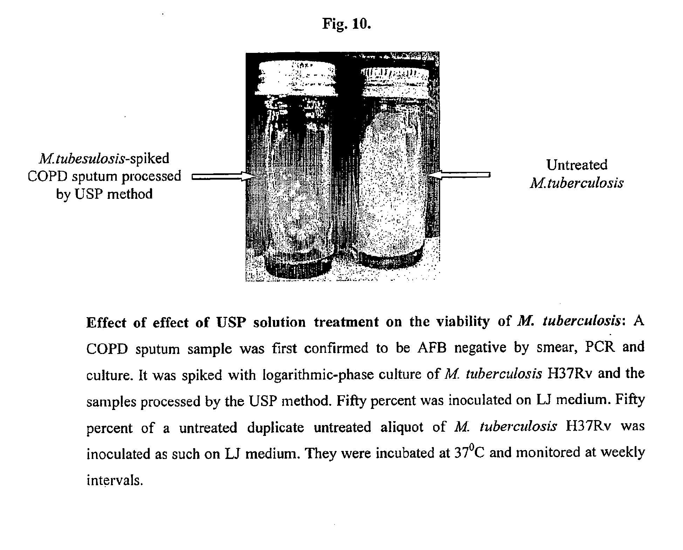

**(i) Lethality of USP solution on *M. tuberculosis* spiked in sputum.** A logarithmic phase *M. tuberculosis* culture was serially diluted up to 10000-fold in duplicate. 10% of one set of dilutions was inoculated onto LJ slant, 100% of each dilution of the duplicate set was spiked into 1ml of sputum aliquots obtained from a patient suffering from COPD. The spiked samples were processed by the USP method and 10% of the processed material was inoculated onto LJ slant. The viable count obtained in the case of untreated cells was 720/ml and that in case of the spiked USP solution treated cells was 400/ml (at 10000-fold dilution) after 8 weeks (Table 3; Fig. 10). Approximately 55% of mycobacteria survived treatment with USP. The CDC method that is currently the most popularly used method for decontamination of clinical samples employs NaOH and with this method, a loss of viability from 28 to 70% has been reported [Krasnow and Wayne. 1966].

**Table 3. Effect of USP on viability of M. tuberculosis cells spiked in COPD sputum.**

| Dilution | Untreated cells (cfu) | USP solution-treated cells (cfu) |
|---|---|---|
| Neat | Confluent | Confluent |
| 1:10 | Confluent | Confluent |
| 1:100 | Confluent | ∼ 500 |
| 1:1000 | ∼ 300 | 134 |
| 1:10000 | 72 | 40 |

However it was found that USP solution containing 4 M GuHCl (in place of 5 M GuHCl) was less harsh on mycobacteria. More viable bacterial colonies were obtained when *M. tuberculosis* was treated with USP containing 4 M GuHCl than with USP having 6M GuHCl); 142 cells/ml in case of cells treated with USP containing 4 M GuHCl and 72 cells /ml in case of cells treated with 6 M GuHCl (at 100 fold dilution) after 4 weeks. The untreated cells of the same dilution corresponded to 277 cells/ml. But it decontaminated clinical samples with equal efficiency. So where culturing of *M. tuberculosis* from clinical samples is of prime importance, USP solution containing 4M GuHCl will increase the chances of culture positivity.
**(ii) Effect of USP on the growth rate of *M. tuberculosis:*** It is well known that treatment with decontaminating reagents like NaOH slows down the growth rate of *M. tuberculosis* from clinical material. A similar observation was made with USP solution-treated bacteria also. USP-treated *M. tuberculosis* colonies appeared after a lag of at least 1-1^{1/2} weeks compared to that of untreated cells on solid media (Middlebrook 7H10 or LJ). NALC-NaOH treated *M. tuberculosis* (CDC method) also grew somewhat faster (visible colonies were obtained at least 4-5 days earlier) than USP-treated bacteria on solid media although no appreciable differences were observed between the two treatments when bacteria were cultured in liquid system BD BACTEC™ MGIT™ 960 System (Becton Dickinson, USA) [see below].
From the above experiments it was estimated that after USP (containing 5 M GuHCl) solution treatment at 37°C for 20 minutes, 56-62 % of the *M. tuberculosis* cells were rendered non-viable. Thus, USP solution is not grossly lethal to the *M. tuberculosis.*
**(iii). The growth rate of *M. tuberculosis* is equivalent after treatment with USP solution and NALC-NaOH in liquid medium (Mycobacterial growth indicator tubes):** It is well known that treatment with decontaminating agents adversely affects the viability of mycobacteria and slows down their growth rate. The effect of USP solution treatment on the growth rate of *M. tuberculosis* was compared to that of NALC-NaOH treatment (CDC method). A logarithmic phase *M. tuberculosis* culture grown in Middlebrook 7H9 liquid medium (with ADC supplement and 0.05% Tween 80) was divided into three sets. One set was treated with USP solution and the other set was treated with NALC-NaOH followed by neutralization with phosphate buffer (CDC protocol). All three sets (i.e., USP solution-treated, NALC-NaOH-treated and untreated) were inoculated into MGIT tubes with PANTA reagent and OADC supplement (Becton Dickinson, USA), and monitored for positive signal using BD BACTEC™ MGIT™ 960 System. Untreated cells gave a positive signal for growth in two days whereas the USP solution-treated cells and NALC-NaOH-treated cells gave a positive signal for growth in five days. This experiment was repeated and the same results were obtained. This suggests that both USP and CDC methods slow down the growth of *M. tuberculosis* in liquid medium (MGIT) to an equal extent.
**(iv)Evaluation of USP culture in a clinical setting.** The USP method of culturing was compared with other conventional methods of culturing (CDC method, modified Pettrof's method and Nassua's method) on a panel of 571 specimens.
Notable findings were
(a). Both the USP and the conventional methods showed comparable percent-positivity. The positivity of the conventional methods (53.6 %) was not significantly higher than that of the USP method (50.1%) and they overlapped at 95% confidence interval.
(b). USP method proved to be very efficient in decontamination and showed lesser number of contaminated cultures (n=5) compared to the conventional methods (n=21) thereby proving it to be more suitable for tropical countries.
(c). USP solution being a neutral pH solution, samples decontaminated with USP solution did not require any neutralization prior to culturing.

Thus in terms of sensitivity, effect on viability and effect on growth rate, the USP method of culture is comparable to the currently accepted methods for the culturing of *M. tuberculosis* from clinical samples.

### (E) PCR.

The PCR test was performed on >700 samples of pulmonary and extra-pulmomary origin.

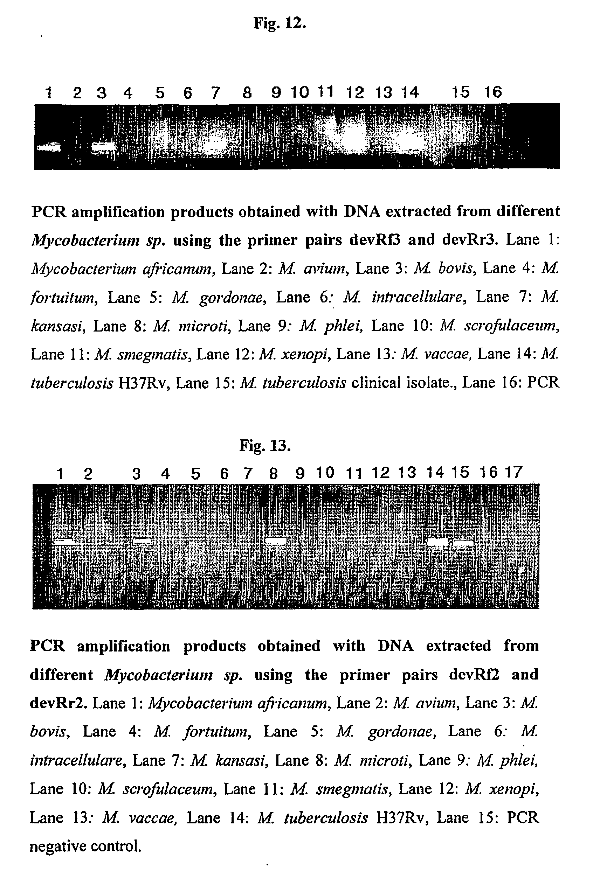

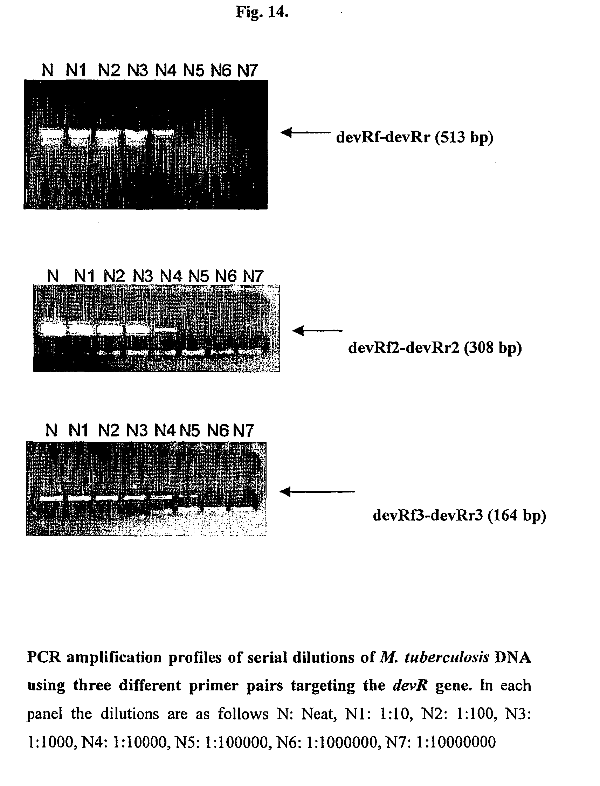

Samples were either fresh or frozen at -20 °C for up to 2 months. Mycobacterial DNA was isolated for this purpose by the USP method. Not a single sample has showed inhibition in the PCR assay indicating that the DNA isolated from the clinical samples by the USP method was free of inhibitors. The PCR assay targets the *devR* gene of *M. tuberculosis* (*Rv 3133c*) and is specific for the organisms of the *M. tuberculosis* complex [Singh *et al*., 1999]. In the published studies, the PCR primer pair devRf and devRr amplified a 513 bp fragment from the target gene [Singh *et al*., 1999; 2000]. It was reasoned that amplification of a smaller region of the same gene was likely to provide equivalent / better sensitivity with paucibacillary and smear negative samples. With this idea in mind two new sets of primers mapping within the *devR* gene were used in order to amplify shorter fragments of the *devR* gene. The new primer pairs used were (i) devRf2 and devRr2 that amplify a 308 bp fragment (Fig. 11) and (ii) devRf and devRr3 that amplify a 164 bp, respectively from the *devR* gene (Fig. 11). The results were compared with the original 513 bp *devR* assay and an IS6110-based assay which is used in a large number of laboratories worldwide.
**(i) Assay specificity.** The specificity of the assays was assessed by the addition of DNA from various mycobacterial species into the PCR reaction. The devRf2 and devRr2 primers showed excellent specificity and a band of expected size (308 bp), was obtained only with DNA from organisms belonging to the *M. tuberculosis* complex and not with DNA from other mycobacterial species (Fig. 13). However, with devRf3 and devRr3 primers, amplification (164 bp product) was obtained with DNA from *M. kansasii* and *M. xenopi* species in addition to *M. africanum, M. bovis* and *M. tuber-culosis.* Further, no amplification was obtained with DNA from *M. microti,* which belongs to the *M. tuberculosis* complex (Fig. 12).
**(ii) Assay sensitivity using purified DNA.** Serial dilutions of pure *M. tuberculosis* DNA were used in PCR assays to assess the limit of detection of the amplified products by ethidium bromide (Table 4, Fig. 14). In comparison to the PCR assay that generated a 513 bp amplification product, the primer pairs devRf2-devRr2 and devRf3-devRr3 showed a marked enhancement in sensitivity. The devRf2 and devRr2 primers [product size 308 bp] showed 2- to 4-fold more sensitivity and the devRf3 and devRr3 primers (product size, 164 bp) showed at least 10-fold more sensitivity over devRf and devRr primers (product size, 513 bp) [Table 4].

**Table 4. Comparative sensitivity of deaR PCR assays using different primer pairs over serial dilution of M. tuberculosis genomic DNA.**

| Amt. of input in PCR assay | | DNA 300ng | 50 ng | 10 ng | 1ng | 100 pg | 10 pg | 5 pg | 2.5 pg | 1 pg | 100 fg |
|---|---|---|---|---|---|---|---|---|---|---|---|
| Bacillary equivalents | | 3×10⁷ | 5×10⁶ | 10⁶ | 10⁵ | 10⁴ | 10³ | 500 | 250 | 100 | 10 |
| Primers | Product size (bp) | | | | | | | | | | |
| devRf/dev Rr | 513 | + | + | + | + | + | + | - | - | - | - |
| devRf2/de vRr2 | 308 | + | + | + | + | + | + | + | +/? | - | - |
| devRf3/de vRr3 | 164 | + | + | + | + | + | + | + | + | + | - |

**(iii)Evaluation of the 'short target' and 'long target' PCR assays in a clinical setting.**

The results of the PCR assays were compared with that of culture and smear microscopy, of 571 samples collected from suspected tuberculosis patients (n = 571) coming to the DOTS Centre at New Delhi Tuberculosis Center, Sunderlal Jain Hospital, Delhi and Tuberculosis research center, Chennai. The assays used were:
1. 'Short target' *devR* PCR assay using a combination of primer pairs devRf2 & devRr2 (product size, 308 bp and devRf3 & devRr3 (product size, 164 bp)(carried out on 571 sputum specimens).
2. 'Long target' *devR* PCR assay using previously published and validated primer pairs devRf and devRr (product size 513 bp) [Singh et al 1998, 2000] (carried out on 506 of the 571 specimens).
3. IS6110 PCR assay carried out on 571 samples using published primers [Eisenach *et al* 1990]. This assay served as a control since it is the most widely used PCR assay worldwide.

The sensitivity of the 'short target' assay was 98.5 %. This was substantially better than that of the 'long target' assay, whose sensitivity was 73.2 %. For example, out of the 571 sputum samples analyzed, 322 sputum samples were positive by the USP smear method and also culture positive. Out of these culture positive and USP smear positive samples, long target assay failed to detect 65 samples, which were detected, by the short target assay confirming the latter assay to be a more sensitive one. Thus the introduction of the 'short target' primers increased the *devR* assay sensitivity by 25.3 % using culture positivity, as gold standard (Data Set 3) and 2-10 folds when compared using serial dilutions of *M. tuberculosis* genomic DNA (Table 4). The overall diagnostic accuracy of the short-target assay was about 7 % higher than that of the long-target assay (Data Set 3).

The enhanced sensitivity of the 'short target' assay was however accompanied by a lower specificity of 77 % as compared to a specificity of 91.9 % with the 'long target' assay (Data set 3). The apparent reduction in specificity of the 'short target' assay is likely a reflection of the enhanced sensitivity of the assay and is unlikely to be due to false positivity/ lower specificity. This is supported by the following:
(i) Out of 328 culture positive samples, the 'short target' PCR detected 323 samples. An equal number were also positive by IS6110 PCR assay. However in this study covering 571 samples, 6 samples were negative by the IS6110 assay while being positive for the *devR* 'short target' assay probably due to absence of IS6110 gene. The IS6110 PCR also detected 2 additional culture positive samples (Data set 3). The IS6110-based PCR assay for amplification of a 123 bp product is specific for organisms of *M. tuberculosis* complex and shows high sensitivity on account of the presence of IS6110 in multiple copies in the genome (as opposed to *devR* which is present in a single copy per genome). But the *devR* short target assay showed comparable sensitivity to IS6110 assay (Data Set 3). The value of *devR* also lies in its universal presence in all *M*. *tuberculosis* isolates in comparison to IS6110 that is reported to be sometimes completely absent or to be present in 1 or few copies in Indian strains (Nacayanan et al 2001). This is reflected in the lower sensitivity of the IS6110 assay in the case of pleural tissue and lymph node specimens in the study with extrapulmonary specimens described later (Data Sets 5 & 6).
(ii) **Non-tuberculosis controls:** Smear microscopy, culture and PCR were carried out on a panel of 78 sputum from 69 patients suffering from diseases clinically proven to be other than tuberculosis. All the samples were negative by smear microscopy, PCR and culture proving the specificity of the assay systems.

### Data set 3

### Sensitivity and specificity of PCR using 'long target' and 'short target' devR PCR).

### Using culture positivity as gold standard; Sample strength, n = 506 for devR-long PCR n = 571 for devR-short PCR.

| | **Culture** | |
|---|---|---|
| **PCR target and result** | Positive | Negative |
| ***DevR*-long (513 bp)** | | |
| Positive | 199 | 19 |
| Negative | 73 | 215 |
| Total | 272 | 234 |
| ***devR*-short(308/164 bp)** | | |
| Positive | 323 | 56 |
| Negative | 5 | 187 |
| Total | 328 | 243 |
| **IS6110 (123 bp)** | | |
| Positive | 325 | 70 |
| Negative | 3 | 173 |
| Total | 328 | 243 |

### Measure of association at 95% confidence interval.

### devR-long:

| | |
|---|---|
| Sensitivity: 73.2 % | (67.4 % - 78.2 %) |
| Specificity: 91.9 % | (87.4 % - 94.9 %) |

| | |
|---|---|
| PPV: 91.3 % | (86.5 % - 94.5 %) |
| NPV: 77.1 % | (69.1 % - 79.5 %) |
| Diagnostic accuracy: 81.8 % | |

### devR-short:

| | |
|---|---|
| Sensitivity: 98.5 % | (96.3 % - 99.4 %) |
| Specificity: 77 % | (71.0 % - 82.0 %) |
| PPV: 85.2 % | (81.8 % - 88.5 %) |
| NPV: 97.4 % | (93.7 % - 99.0 %) |
| Diagnostic accuracy: 89.32 % | |

### IS6110:

| | |
|---|---|
| Sensitivity: 99.1 % | (97.1 % - 99.8 %) |
| Specificity: 71.2 % | (65 % - 76.7 %) |
| PPV: 82.28 % | (78.1 % - 85.8 %) |
| NPV: 98.3 % | (94.7 % - 99.6 %) |
| Diagnostic accuracy: 87.22 % | |

**Extrapulmonary samples:** The USP method was evaluated for its performance in the diagnosis of tuberculous pleural effusion and lymphadenopathy in a blinded study with coded pleural fluid, pleural tissue and lymph-node biopsy specimens collected from the patients coming to the OPD of Safdarjung Hospital, New Delhi, India. The samples comprised of 100 specimens from 88 patients including 77 specimens from TB patients and 23 specimens from control subjects with diseases like malignancy, amoebiasis, CHF, Sarcoidosis and reactive lymphadenopathy (nonspecific inflammation). Inclusion criteria was based on any one or more of the following parameters:
a) Histopathology/Cytopathology consistent with TB (b) Positive smear for AFB (c) Culture positive for *M. tb.* (d) Clinical response to ATT. Specimens were processed by the USP method and smear microscopy, culture and PCR were performed. In this study a comparison between *devR* and IS6110 based PCR was carried out. *devR* PCR was carried out with the primer pairs devRf3/devRr3
   *M. tuberculosis* DNA that was isolated from the specimens was of good quality and no PCR inhibition was noticeable. The USP method of smear microscopy detected eight specimens (6 pleural fluid and one each of lymph node and pleural tissue biopsy) (Data Set 4) as positive against three with the conventional method of smear microscopy. The sensitivity of USP smear microscopy was 10.4% in this group of extrapulmonary specimens whereas that of the conventional method was only 3.9 %. Thus the USP methods enabled improved conclusive AFB smear microscopy from tissue biopsy specimens and other extrapulmonary specimens, compared to routine diagnostic methods.
   Five specimens were positive by the USP method of culturing (sensitivity 6.5 %)(Data Set 4). The *devR* PCR showed better sensitivity with pleural tissue and lymph node (100 % and 75 % respectively) specimens than IS6110, (75 % and 50 % respectively) probably due to absence of any copies of IS6110 in these strains (Data Sets 5 & 6). Thus the USP method of specimen processing and *devR* PCR served as a useful modality to arrive at the definitive diagnosis of tuberculous pleural effusion and lymphadenopathy.

### Data Set 4:

### Smear microscopy and culture status of extrapulmonary specimens processed by the USP method

| **Sample Type** | **Total number** | **Smear positive** | **Culture positive** |
|---|---|---|---|
| **Pleural fluid** | **69** | **6** | **4** |
| **Pleural tissue** | **11** | **1** | **0** |
| **Lymph node** | **20** | **1** | **1** |

### Data Set 5:

### Sensitivity and specificity values (%) for devR- short target PCR for the extrapulmonary samples

| | **Pleural Fluid** | **Pleural tissue** | **Lymph node** |
|---|---|---|---|
| **Sensitivity (%)** | **80.9** | **100** | **75** |
| **Specificity (%)** | **93.3** | **100** | **66.7** |
| **PPV (%)** | **97.1** | **100** | **90** |
| **NPV (%)** | **63.6** | **100** | **40** |
| **Efficiency (%)** | **84.2** | **100** | **73.3** |

### Data Set 6:

### Sensitivity and specificity values (%) for IS6110 PCR for the extrapulmonary samples.

| | **Pleural Fluid** | **Pleural tissue** | **Lymph node** |
|---|---|---|---|
| **Sensitivity (%)** | **93** | **75** | **50** |
| **Specificity (%)** | **96.15** | **100** | **75** |
| **PPV (%)** | **97.6** | **100** | **88.9** |
| **NPV(%)** | **89.3** | **60** | **27.3** |
| **Efficiency (%)** | **94.2** | **81.9** | **55** |

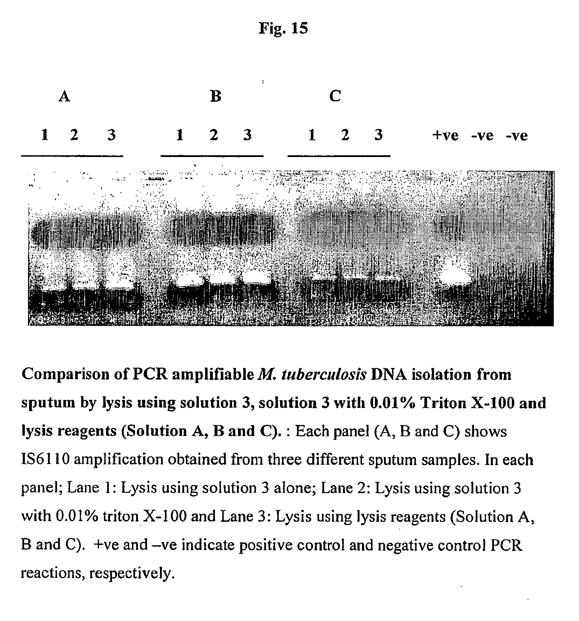

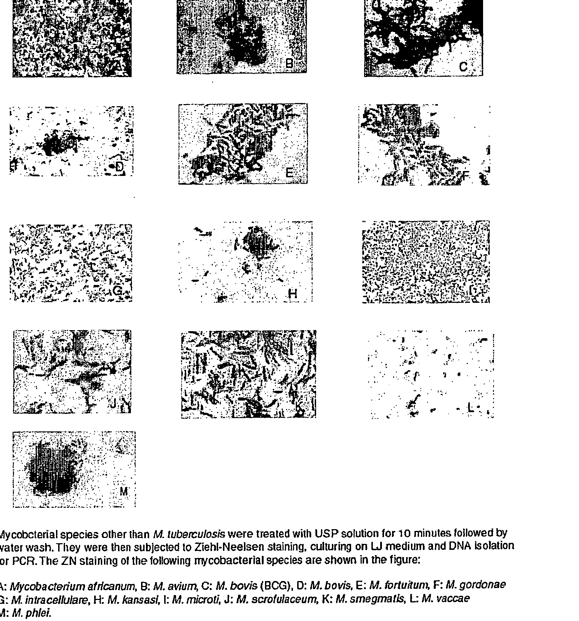

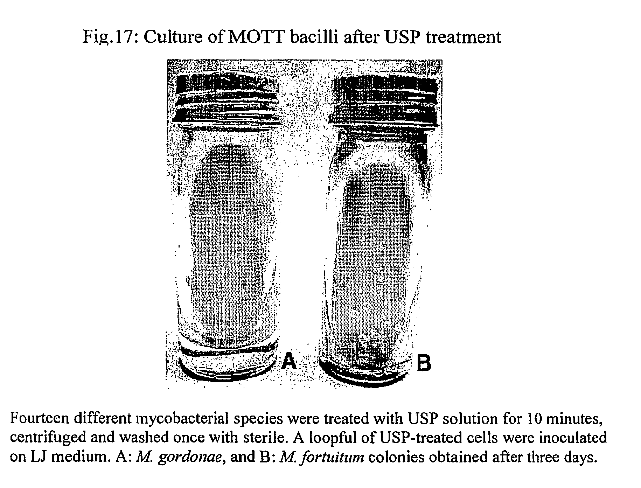

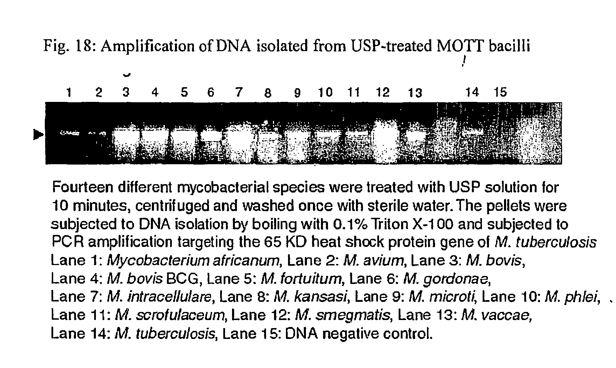

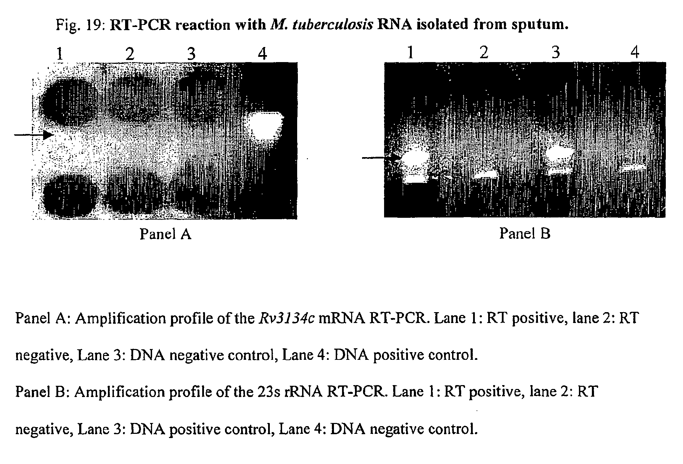

The statistical analysis was done using SAS 8.0 software (SAS Institute Inc. Cary, NC, USA.). Descriptive statistics, frequency distribution and percentage of the variables have been calculated. To evaluate diagnostic accuracy and reliability of the tests in comparison to gold standards, sensitivity, specificity, positive and negative predictive values and their confidence intervals at 95 % have been calculated.
**(F) Simplified protocol for lysis of mycobacteria present in the processed specimen prior to DNA isolation for PCR:** After USP processing if the pellet size is drastically reduced so as to be nearly invisible or is very minute or if the sample is a high bacillary load sample, lysis may be carried out by heating the resuspension directly in solution 3 or after addition of Triton X-100 (final concentration of 0.01 %) at 90-100 °C for 30-40 minutes. Three AFB positive sputum specimens were processed by the USP method. The processed pellet was resuspended into resuspension solution (solution 3) and divided into three equal aliquots for each specimen. One aliquot was pelleted and to the pellet was added lysis reagents (namely, solutions A, B and C), to the second aliquot was added Triton X-100 at a final concentration of 0.01 % and the other was kept as such. All the three were boiled at 90°C for 40 minutes and PCR was carried out with the supernatant. All the three lysates in each of the three specimens yielded amplicons with almost equal intensity when analysed by agarose gel electrophoresis (Fig. 15). These results indicate that in case of very clean pellet obtained after processing or for samples with high bacillary loads, the addition of the lysis reagents (namely, solutions A, B and C) may be avoided; but for best results, it is recommended that the lysis reagents (Solution A, B and C) be used for isolation of PCR-amplifiable DNA.
**(G) USP method is applicable to diagnosis of mycobacteriosis caused by Mycobacterium other than tuberculosis type (MOTT) as it does not have any detrimental effect on them:** MOTT bacilli were subjected to USP treatment for 10-15 minutes at room temperature, followed by a wash with sterile triple distilled water. Each species was then subjected to smear microscopy, culture on LJ slant and PCR to check for detrimental effect of USP solution, if any. The MOTT species used were: *M. avium, M, fortuitum, M. gordonae, M. intracellulare, M. kansasi, M. scrofulacem, M. smegmatis, M. phlei, and M. vaccae.* Organisms belonging to the *M. tuberculosis* complex like *M*. *africanum, M. bovis, M. bovis*(BCG), *M. microti* were also included in the study. All the organisms retained their AFB status (Fig. 16) and viability. However *M. smegmatis* though it maintained its integrity and AFB property, lost its viability. Fig.17 shows two USP-treated rapid growers. Good quality PCR-amplifiable DNA was isolated from all the USP-treated Mycobacterium (Fig. 18).
**(H) Samples processed by the USP method are compatible with procedures for RNA isolation from *M. tuberculosis:***
   The USP method is also compatible with the isolation of high quality *M. tuberculosis* RNA from sputum specimens. For this purpose USP solution with the following composition was used.6M Guanidinium hydrochloride, 50 mM Tris-Cl, pH 7.5, 25 mM EDTA, 0.5% Sarcosyl and 0.2 M β- mercaptoethanol.
   The sputum specimen used was graded 3+ by the direct method of smear microscopy. To the specimen (5-7 ml), was added 1.5 times the volume of USP solution and the mixture was homogenized. To it was added 10 ml of sterile DEPC water and mixed. It was then centrifuged at 5,000 rpm for 10 minutes at room temperature. The pellet was again washed with 2-3 ml of USP solution followed by a wash with sterile DEPC water. The final processed pellet can be used as a starting material for RNA isolation by an appropriate means. For example, using the Qiagen Rneasy Mini Kit as per manufacturer's protocol. The isolated *M. tuberculosis* RNA was subjected to reverse transcription reaction using Stratrascript reverse transcriptase (RT) enzyme (Stratagene, USA) to obtain cDNA. The cDNA was subjected to amplification using *M. tuberculosis* rRNA-(23S rRNA) and mRNA (*Rv 3134c* gene)-specific primers to detect the presence of *M. tuberculosis* RNA. RT negative controls in which the isolated RNA was subjected to reverse transcription reaction without the reverse transcriptase enzyme were also run for PCR to monitor for the presence of any contaminating genomic DNA in the preparation. Amplifications corresponding to both the ribosomal and messenger cDNAs were obtained (Fig. 19) proving that both classes of *M. tuberculosis* RNA was isolated from the sputum specimen.
**(I) Effect of USP solution on the viability and staining property of Gram staining** bacteria: The effect of USP solution on the viability and staining properties of *Escherichia coli, Pseudomanas sp.,* and *Staphylococcus sp.,* the three most common microflora present in sputum specimens, was evaluated. The bacteria were treated with solution 1 for 10-15 minutes at room temperature followed by centrifugation and washing of the pellet with sterile water as described. The pellets were then resuspended in solution 3 and 10% was smeared in glass slides (in duplicates) and 40% was inoculated in nutrient agar media and incubated at 37°C to monitor for any growth. This was followed by Gram staining of the slides. Gram staining was also performed on the untreated bacteria.

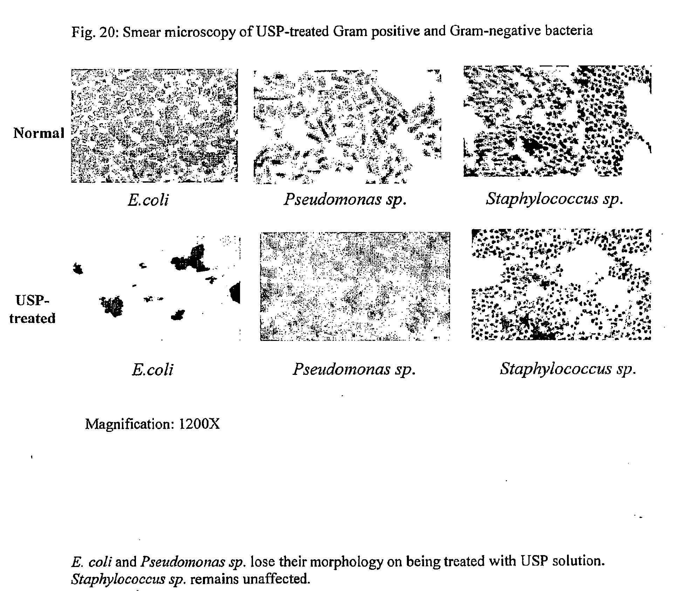

It was observed that both *Pseudomonas sp.* and *E. coli,* were lysed by treatment with USP solution as evident from the total loss of morphology as seen by Gram staining (Fig. 20) and absence of any growth in the nutrient agar media even after one week. However *Staphylococcus sp.* retained its morphology as well as viability (checked by growth on nutrient-agar medium) on treatment with USP solution. However it did not grow in LJ media possibly due to selectivity of the media when a sputum sample containing both *M. tuberculosis* and *Staphylococcus sp.* was processed by the USP method and cultured on LJ media (not shown).

So USP solution lyses and kills Gram negative bacteria but does not have any detrimental effect on Gram positive bacteria like *Staphylococcus sp.* and hence can also be used for diagnosis of staphylococcal disease.

### 8. Main advantages of the invention:

(i) A methodology is developed that involves optimized sample processing using a novel use of a chaotropic agent to provide rapid, sensitive and accurate diagnosis of tuberculosis. The unique properties of the mycobacterial cell wall render mycobacteria selectively resistant to the action of guanidinium hydrochloride.
(ii) In addition to *M. tuberculosis* and *M*. *bovis,* samples containing other mycobacteria can also be effectively processed by the USP method prior to subjecting the material to diagnostic tests.
(iii) The claimed technology is modular; it is eminently suited for highly sensitive smear microscopy, culture and isolation of good quality inhibitor-free mycobacterial DNA and RNA for use in nucleic acid-based diagnostic assays. Further, a sensitive PCR assay is described that enables sensitive and specific diagnosis of tuberculosis.
(iv) The methodology being relatively simple and user-friendly can be performed by technicians having minimal scientific background and training.

### Advantages of USP Smear Microscopy.

1. The major advantage of the USP method over the existing methods of smear microscopy is its very high sensitivity. The method has been shown to reproducibly detect samples containing up to 300-400 bacilli/ml of sputum. This is ∼ 30 fold more sensitive than the direct method of smear examination. The sensitivity (limit of detection) can be further increased by using more than the prescribed 10% of the processed specimen for smear microscopy.
2. Since the method deals with the whole sputum sample, samples that lack purulence or contain nasopharyngeal discharge or saliva can also be processed easily by this method.
3. Since tissue and cell debris and the proteinaceous waste in the sputum are effectively removed, the smear can be made in a minimal area (1 cm × 1 cm). Up to 10% of the sample may be used with no chance of getting an excessively thick smear or obtaining a high background that interferes with the microscopic examination of the slides (see Fig. 2).
4. In case culture and PCR tests are not performed, > 10% of the sample can be examined on multiple slides. This approach is expected to further improve the sensitivity of smear microscopy by USP method.
5. This method is particularly beneficial while preparing smears from tissue biopsy samples. It can bypass routine histopathological procedures for preparing smears from solid biopsy specimens for AFB smear microscopy. This method yields high quality smears from tissue biopsy samples suitable for AFB staining, which is not possible either by the direct or concentration (CDC) methods of smearing.
6. The method is also compatible with mycobacteria other than *M. tuberculosis.*

### Advantages of USP Culture.

1. The process does not involve the use of costly reagents like NALC.
2. The USP solution is a buffered solution having neutral pH. So neutralization of the sample is not necessary before culturing.
3. The process is versatile and can be used for culturing *M. tuberculosis* from a wide range of pulmonary and extra-pulmonary samples in addition to sputum.
4. The USP solution serves as a very effective decontaminant and is suitable for use in tropical countries with humid climate.
5. The method is also compatible with culture of mycobacteria other than *M. tuberculosis* and Gram positive organisms such as *Staphylococcus sp.*

### Advantages of USP Nucleic acids isolation method.

1. This method being a multipurpose method, a separate method is not required for isolating DNA/RNA from the samples processed for smear microscopy and culture.
2. It can isolate inhibitor-free PCR-amplifiable DNA from a wide range of samples of pulmonary and extra-pulmonary origin, in fact it is claimed to be a universal processing method. It efficiently removes hemoglobin from clinical samples (eg. Blood, hemoptyic sputum samples) which is known to be a potent PCR inhibitor (Mahony *et al.* 1998; Al-Soud and Radstrom P 2001; Kang *et al.* 2002).
3. It does not involve the use of hazardous organic solvents like phenol or chloroform or expensive enzymes like lysozyme, etc.
4. Since it contains chaotropic agent GuHCl, a potent RNase denaturant, it is compatible with high quality mycobacterial RNA isolation from clinical specimens.

### Advantages of devR-based PCR.

1. The *devR* gene is conserved amongst all species and isolates of *M. tuberculosis* complex including *M. tuberculosis* and *M. bovis* in contrast to a popular assay based on insertion sequence IS6110. IS6110 sequences have been reported to be missing in some proportion of clinical isolates of India and worldwide. [Dale *et al.* 1997, Barlow *et al.* 2001, Narayanan *et al.* 2001].

### Advantage of Modular technology.

This technology is suitable for any kind of body fluid and tissue biopsy (except for fecal matter which has not been tested).The claimed technology can be used in a variety of clinical settings and laboratories. (1) It can be used for smear microscopy in laboratories having minimal equipment (only a vortex shaker, light microscope and ambient temperature centrifuge that can go up to a speed of 4000 rpm). (2) It can be used for smear microscopy and culture in laboratories having additional facilities for mycobacterial culture. (3) Finally PCR and RT-PCR tests can be applied in sophisticated laboratories.

The technology thus can be marketed as the following to suit individual requirements:
Kit 1: Universal sample processing (USP) kit [containing 4-6 M GuHCl and 0.1-0.2 β-mercaptoethanol; see "Detailed description" for details].
Kit 2: USP, smear and culture kit.
Kit 3: USP, smear, culture and DNA isolation kit.
Kit 4: Complete kit (USP, smear, culture, DNA isolation and PCR).
Kit 5: As a part of mycobacterial RNA isolation kit from clinical specimens.

### 9. Various significant aspects of the disclosure

1. This method alone serves all the three important aspects of TB diagnosis by molecular and microbiological methods, namely smear microscopy, culture and PCR/RT-PCR. In fact it is claimed to be a universal processing method.
2. The methodology is versatile and applicable on all types of human clinical material including respiratory specimens (spontaneously expectorated sputum, normal saline-nebulized induced sputum, transtracheal aspirate, bronchoalveolar lavage, laryngeal swab, nasopharyngeal swab), body fluids (pleural fluid, pericardial fluid, joint aspirate, gastric aspirate, peritoneal fluid, cerebrospinal fluid, urine, pus, endometrial aspirate, synovial fluid), body tissues (blood, bone marrow biopsy) and solid organs (lymph node, bone, skin) and bovine samples (lymph gland, milk and blood).
3. The method is rapid; it requires a maximum of 2 hours for complete processing of a sample.
4. The method is also suitable for smear microscopy, culture and PCR from frozen sputum samples (stored at -20 °C for up to 2 months) and frozen extra pulmonary samples.
5. The method is suitable for the isolation of host (human) DNA with certain modifications.

### Other aspects relating to USP solution.

**1. Safe:** (i) USP solution contains guanidinium hydrochloride (GuHCl) instead of guanidinium isothiocyanate (GuSCN) as the principal component. Since GuSCN yields a potentially toxic gas HCN on contact with acids, extreme care is to be taken while disposing off solutions containing this chemical. Therefore it is not suitable for unspecialized and routine microbiological laboratories. But the use of GuHCl in this reagent makes it safe to handle and dispose. (ii) It does not use any hazardous organic solvents.
**2. Stability and handing:** No special handling required.
**3. Cost effective:** The USP method does not involve the use of the expensive reagent NALC unlike the IRS method. Further, GuHCl costs lesser than GuSCN which is a part of inhibitor removal solution.
**4. USP solution is a neutral pH solution:** The USP solution is a buffered solution of a neutral pH. Therefore no additional step involving neutralization is necessary before culturing of *M. tuberculosis* and other mycobacteria from the decontaminated samples. USP solution is also compatible with RNA isolation protocols.
5. Mucolytic, decontaminating, protein denaturing, chaotropic, liquefying, tissue softening/digesting, mycobacteria-releasing action.
6. Synergistic action of the three main components of USP: including chaotrope GuHCl, detergent sarcosyl and reducing agent β-mercaptoethanol.

### Aspects relating to smear microscopy.

1. The method is eminently suitable for the preparation of smears for highly sensitive microscopy. The method has been shown to reproducibly detect samples containing up to 300-400 bacilli/ml of sputum. This is ∼ 30 fold more sensitive than the direct method of smear examination. The sensitivity (limit of detection) can be further increased by using more than the prescribed 10% of the processed specimen for smear microscopy.
2. Since the method deals with the whole sputum sample, samples that lack purulence or contain nasopharyngeal discharge or saliva can also be processed easily by this method.
3. Since this method effectively removes the tissue and cell debris and the proteinaceous waste in the sputum (counterstaining material), the smear can be made in a minimal area (1 cm × 1 cm) taking at least 10% of the sample with no chances of getting an excessively thick smear or obtaining a high background which interferes with the microscopic examination of the slides (see Fig. 9B). This enables more bacilli to be smeared on the slide thereby increasing the sensitivity of the microscopic detection method considerably and minimizing the time spent in examining every slide. Thus the USP method generally converts slides that are graded as 1+ or scanty by the direct method to 2+/3+ or 2+/1+, respectively. Following the same principle, the slides that are read as negative by the direct method become positive by the USP method. It must be mentioned here that the number of fields scanned in case of negative samples for each method of smearing was 300-500 and was not limited to 100 fields to be sure of the status of the samples. To the best of our knowledge, this method is the most sensitive method reported till date, having a detection limit of 300-400 bacilli per ml of sample. (Fig. 3).
4. The sensitivity (limit of detection) can be further increased by using more than the prescribed 10% of the processed specimen for smear microscopy.
5. In case culture and PCR tests are not performed, > 10% of the sample can be examined on multiple slides. This approach is expected to further improve the sensitivity of smear microscopy by USP method.
6. The method is also compatible with mycobacteria other than *M. tuberculosis.*
7. The method of smear microscopy is applicable on all mycobacteria including slow growing species like organisms belonging to the *M. tuberculosis* complex, *M. avium, M. intracellulare, M. paratuberccslosis M. xenopi,* etc. rapid growing species like *M. smegmatis, M, fortuitum, M. phlei, M. vaccae, M. gordonae* etc. and non-cultivable species like *M. leprae*.
8. The USP smear microscopy method is compatible with Gram positive organism such as Staphylococcus sp and is predicted to be applicable on other acid -fast bacilli (e.g. *Cryptosporidium sp., Nocardia sp., Isospora belli* and *Rhodococcus equi*.)
9. Highly sensitive: The process effectively removes the background counterstaining material from the samples, enabling smearing of at least 10% of the sample on the slide, which would have been impossible otherwise. Owing to such efficient removal of the counterstaining junk, smearing as much as 10% of the sample also does not produce a thick smear and the smear can be very easily heat-fixed and stained (Fig. 9b).
10. Tissue biopsy samples can be directly processed for smear microscopy bypassing the routine histologic procedures of fixing, paraffin-embedding and sectioning.

### Aspects relating to Culture.

1. The USP solution serves as a very effective decontaminant and is suitable for use in tropical countries with humid climate.
2. The process does not involve the use of costly reagents like NALC.
3. The USP solution is a buffered solution having neutral pH and does not involve the use of NaOH. So neutralization of the sample is not necessary before culturing. Sample neutralization is a prime requirement in methods involving decontamination with NaOH (such as CDC method). Improper neutralization often adversely affects culture results.
4. The process is versatile and can be used for culturing *M. tuberculosis* from a wide range of pulmonary and extra-pulmonary samples in addition to sputum.
5. The method is also compatible with culture of MOTT bacilli.

### Aspects relating to DNA isolation and devR-based PCR.

1. This method being a multipurpose method, no separate method is needed for isolating DNA from the samples processed for smear microscopy and culture.
2. This method efficiently removes PCR inhibitors. Inhibitor-free PCR-amplifiable DNA can be reproducibly isolated from all types of pulmonary and extra-pulmonary samples.
3. It does not involve the use of hazardous organic solvents like phenol or chloroform or expensive enzymes like lysozyme; etc.
4. The DNA isolation method would be compatible with nucleic acid amplification methods other than PCR such as ligase chain reaction, strand displacement assay, etc.
5. A specific and sensitive PCR assay is described that enables sensitive and specific diagnosis of tuberculosis.
6. Compared to the IRS method of DNA isolation [Chakravorty and Tyagi 2001; Inhibitor removal solution (IRS) is covered under patent application entitled "A rapid, efficient, single - tube extraction procedure for the isolation of PCR - amplifiable *M.tuberculosis* DNA from clinical specimens", filed in Patents Office, New Delhi. Appln. No. 497/DEL/2000.], the USP method is much simplified and involves lesser technology. Firstly, the centrifugation can be performed in table-top centrifuges which can go up to a maximum speed of 4000 rpm and at room temperature, thereby avoiding the necessity of a high speed refrigerated centrifuge. Secondly, the use of NALC as a mucolytic agent, is eliminated, thereby saving on cost. Thirdly, the USP method is environment friendly as it does not involve the use of guanidinium isothiocyanate. Fourthly, the USP method does not require the use of lysozyme enzyme.
7. The DNA isolation method is also compatible with nucleic acid amplification methods employed for the detection of other mycobacteria.
8. Since the USP processing method does not involve treatment with alkali, it is also compatible with RNA isolation procedures from clinical specimens and RNA amplification procedures such as transcription-mediated amplification.

### Aspects relating to RNA isolation and amplification techniques:

1. USP-treated mycobacteria are compatible for use with standard RNA-isolation protocols.
2. RNA isolated thereby are suitable for further analysis by amplification techniques employing enzymes such as reverse transcriptase, Taq DNA polymerase etc.
3. Both mRNA and rRNA are successfully isolated from USP-treated *M. tuberculosis.*

### References:

1. Akhtar M, Bretzel G, Boulahbal F, Dawson D, Fattorini L, Feldmann K, Frieden T, Havelková M .N de Kantor I , Kim S J, Küchler R, Lamothe F, Laszlo A, Casabona NM, McDougall AC, Miömer H, Orefici G, Paramasivan CN, Pattyn SR, Reniero A, Rieder HL, Ridderhof J, Rüsch-Gerdes S, Siddiqi SH, Spinaci S, Urbanczik R, Vincent V, Weyer K. (2000) Technical guide: Sputum Examination For Tuberculosis by Direct Microscopy in Low Income Countries 5th edition, International Union Against Tuberculosis and Lung Disease ,68 boulevard Saint Michel, 75006 Paris, France.
2. Al-Soud WA, Radstrom P. (2001) Purification and characterization of PCR-inhibitory components in blood cells. J Clin Microbiol 39:485-93.
3. Barlow RE, Gascoyne-Binzi DM, Gillespie SH, Dickens A, Qamer S, Hawkey PM. (2001) Comparison of variable number tandem repeat and IS6110-restriction fragment length polymorphism analyses for discrimination of high- and low-copy-number IS6110 Mycobacterium tuberculosis isolates. J Clin Microbiol 39:2453-7.
4. Bruchfeld J, Aderaye G, Palme IB, Bjorvatn B, Kallenius G, Lindquist L. (2000) Sputum concentration improves diagnosis of tuberculosis in a setting with a thigh prevalence of HIV. Trans R Soc Trop Med Hyg 94:677-80.
5. Centres for Disease Control. US Department of Health and Human Services, In Kent PT and Kubica GP editors. (1995) Public health mycobacteriology: A guide for the level III laboratory, Centres for Disease Control, Atlanta.
6. Chakravorty S and Tyagi JS. (2001) Novel use of Guanidinium isothiocyanate in the isolation of Mycobacterium tuberculosis DNA from clinical material. FEMS Microbiol Lett 205:113-7.
7. Dale JW, Tang TH, Wall S, Zainuddin ZF, Plikaytis B. (1997) Conservation of IS6110 sequence in strains of Mycobacterium tuberculosis with single and multiple copies. Tuber Lung Dis 78:225-7.
8. de Lamballerie X, Zandotti C, Vignoli C, Bollet C and de Micco P. (1992) A one-step microbial DNA extraction method using "Chelex-100" suitable for gene amplification. Res Microbiol 143:785-790.
9. Eisenach KD, Cave MD, Bates JH and Crawford JT (1990) Polymerase chain reaction amplification of repetitive DNA sequence specific for Mycobacterium tuberculosis. J Infect Dis 161:977-981.
10. Farnia P, Mohammadi F, Zarifi Z, Tabatabee DJ, Ganavi J, Ghazisaeedi K, Farnia PK, Gheydi M,Bahadori M, Masjedi MR, and Velayati AA (2002) Improving Sensitivity of Direct Microscopy for Detection of Acid-Fast Bacilli in Sputum: Use of Chitin in Mucus Digestion. J Clin Microbiol 40:508-511.
11. Garay JE (2000) Analysis of a simplified concentration sputum smear technique for pulmonary tuberculosis diagnosis in rural hospitals. Trop Doct 30:70-2.
12. Gebre N, Karlsson U, Jonsson G, Macaden R, Wolde A, Assefa A, Miomer H. (1995) Improved microscopical diagnosis of pulmonary tuberculosis in developing countries. Trans R Soc Trop Med Hyg 89:191-3.
13. Githui W, Kitui F, Juma ES, Obwana DO, Mwai J, Kwamanga D. (1993) A comparative study on the reliability of the fluorescence microscopy and Ziehl-Neelsen method in the diagnosis of pulmonary tuberculosis. East Afr Med Journal 70(5).
14. Kang EY, Choi JA, Seo BK, Oh YW, Lee CK, Shim JJ. (2002) Utility of polymerase chain reaction for detecting Mycobacterium tuberculosis in specimens from percutaneous transthoracic needle aspiration. Radiology 225:205-9.
15. Kent, PT and Kubica GP. (1995). Public health mycobacteriology. A guide to the level III laboratory. U.S. Department of Health and Human Services, Public Health Service, Centers for Disease Control, Atlanta, Ga.
16. Koneman EW, Stephen DA, William MJ, Schrenkenberger P and Winn WC Jr. (1997) Editors. Color Atlas and Textbook of Diagnostic Microbiology, Lippincott publications, Philadelphia, New York. 5th Edition, Chapter 17,pp 893-952.
17. Krasnow I and Wayne LG. (1966). Sputum digestion. I. The mortality rate of tubercle bacilli in various digestion systems. Am J Clin Pathol 45:352-355.
18. Mahony J, Chong S, Jang D, Luinstra K, Faught M, Dalby D, Sellors J, Chernesky M. (1998) Urine specimens from pregnant and nonpregnant women inhibitory to amplification of Chlamydia trachomatis nucleic acid by PCR, ligase chain reaction, and transcription-mediated amplification: identification of urinary substances associated with inhibition and removal of inhibitory activity. J Clin Microbiol 36:3122-6
19. Narayanan S, Parandaman V, Narayanan PR, Venkatesan P, Girish C, Mahadevan S, Rajajee S. (2001) Evaluation of PCR using TRC(4) and IS6110 primers in detection of tuberculous meningitis. J Clin Microbiol 2001 39:2006-8
20. Noordhoek GT, Kaan JA, Mulder S, Wilke H and Kolk AHJ. (1995) Routine application of the polymerase chain reaction for detection of Mycobacterium tuberculosis in clinical samples. J. Clin. Pathol. 48:810-814.
21. Perera J, Chandrasekharan NV and Karunanayake EH. (1994) PCR based detection of Mycobacterium tuberculosis: Effect of sample preparation. Southeast Asian J Trop Med Pub Health. 25: 693-697.
22. Perera J, Arachchi DM. (1999) The optimum relative centrifugal force and centrifugation time for improved sensitivity of smear and culture for detection of Mycobacterium tuberculosis from sputum. Trans R Soc Trop Med Hyg 93:405-9.
23. Perkins MD. (2000) New diagnostic tools for tuberculosis. Int J Tuberc Lung Dis 4;S182-S188.
24. Selvakumar N, Rahman F, Garg R, Rajasekaran S, Mohan NS, Thyagarajan K, Sundaram V, Santha T, Frieden TR, Narayanan PR. (2002) Evaluation of the phenol ammonium sulfate sedimentation smear microscopy method for diagnosis of pulmonary tuberculosis. J Clin Microbiol 40:3017-20.
25. Singh KK, Nair MD, Radhakrishnan K and Tyagi JS. (1999) Utility of PCR assay in diagnosis of En-plaque Tuberculoma of the brain. J Clin Microbiol: 467-470.
26. Singh KK, Muralidhar M, Kumar A, Chattopadhyaya TK, Kapila K, Singh MK, Sharma SK, Jain NK and Tyagi JS. (2000) Comparison of in house polymerase chain reaction with conventional techniques for the detection of Mycobacterium tuberculosis DNA in granulomatous lymphadenopathy. J Clin Pathol 53:355-361.
27. Thornton CG, MacLellan KM, Brink, Jr TL, Lockwood DE, Romagnoli M, Turner J, Merz WG, Schwalbe RS, Moody M, Lue Y, and Passen S. (1998) Novel Method for Processing Respiratory Specimens for Detection of Mycobacteria by Using C18-Carboxypropylbetaine: Blinded Study. J Clin Microbiol 36:1996-2003.
28. Van Deun A, Maug AK, Cooreman E, Hossain MA, Chambuganj N, Rema V, Marandi H, Kawria A, Portaels F. (2000) Bleach sedimentation method for increased sensitivity of sputum smear microscopy: does it work? Int J Tuberc Lung Dis 4:371-6.
29. Walsh PS, Metzger DA and Higuchi R. (1991) Chelex-100 as a medium for simple extraction of DNA for PCR-based typing from forensic material. Biotechniques. 10:505-513.
30. Wilkinson D, Sturm AW. (1997) Diagnosing tuberculosis in a resource-poor setting: the value of sputum concentration. Trans R Soc Trop Med Hyg 91:420-1.
31. www.tbcindia.org

## Claims

1. A set of primers devRf3 and devRr3 consisting of SEQ ID No. 3 and SEQ ID No. 4, respectively.

2. A method of using primers of SEQ ID No. 3 and SEQ ID No. 4 of gene *devR* for screening patients of tuberculosis, said method comprising the steps of conducting Polymerase Chain Reaction (PCR) on DNA or RNA of a sample from a subject using said primers, and identifying the subjects suffering from tuberculosis.

## Patentansprüche

1. Satz von Primern devRf3 und devRr3, die aus SEQ ID Nr. 3 bzw. SEQ ID Nr. 4 bestehen.

2. Verfahren zur Verwendung von Primern von SEQ ID Nr. 3 und SEQ ID Nr. 4 des Gens *devR* zum Screenen von Patienten auf Tuberkulose, wobei das Verfahren die Schritte des Durchführens einer Polymerasekettenreaktion (PCR) an DNA oder RNA einer Probe von einem Probanden unter Verwendung der Primer und des Identifizierens der Probanden, die an Tuberkulose leiden, umfasst.

## Revendications

1. Ensemble d'amorces devRf3 et devRr3, constitué de la SEQ ID N° 3 et de la SEQ ID N° 4, respectivement.

2. Procédé d'utilisation d'amorces de SEQ ID N° 3 et de SEQ ID N° 4 du gène *devR* pour le dépistage de la tuberculose chez des patients, ledit procédé comprenant les étapes consistant à réaliser une réaction en chaîne par polymérase (PCR) sur de l'ADN ou de l'ARN d'un échantillon provenant d'un sujet en utilisant lesdites amorces, et à identifier les sujets atteints de tuberculose.


### SEQUENCE LISTING

*<110> All India Institute of Medical Science*
*<120> A method for diagnosis of tuberculosis by smear microscopy, culture and polymerase chain reaction us ing processed clinical samples and kit thereof.*
*<130> PCT-351*
*<160> 4*
*<170> PatentIn version 3.1*
*<210> 1*
   *<211> 20*
   *<212> DNA*
   *<213> Artificial sequence*
*<400> 1*
   *tggcaacggc attgaactgt 20*
*<210> 2*
   *<211> 19*
   *<212> DNA*
   *<213> Artificial sequence*
*<400> 2*
   *taagcaggcc cagtagcgt 19*
*<210> 3*
   *<211> 19*
   *<212> DNA*
   *<213> Artificial sequence*
*<400> 3*
   *atctgttgtc ccgcatgcc 19*
*<210> 4*
   *<211> 19*
   *<212> DNA*
   *<213> Artificial sequence*
*<400> 4*
   *gtrcagcgcc cacatcttt 19*